# DEMANDE DE BREVET EUROPEEN

(11) **EP 0 957 168 A1**
(43) Date de publication de la demande: **17.11.1999**
(21) Numéro de dépôt: 98201310.4
(22) Date de dépôt: 22.04.1998
(51) Int. Cl.: C12N 15/52, C12P 19/18, C12N 9/10, C08B 37/00, C12Q 1/68, C12N 1/21

(54) **Identification de gènes de Streptococcus thermophilus Sfi39 impliqués dans la biosynthèse d'exopolysaccharides**

(71) Demandeur: SOCIETE DES PRODUITS NESTLE S.A., 1800 Vevey (CH)
(72) Inventeur: Stingele, Francesca, 1018 Lausanne (CH); Germond, Jacques Edouard, 1023 Crissier (CH)
(74) Mandataire: Van Malderen, Joelle

(57) **Abrégé**

Enzyme recombinée, susceptible d'être impliquée dans la biosynthèse de l'EPS ayant l'unité saccharidique répétitive 
β-D-Galp1
↓
6
→3)-β-D-Glcp-(1→3)-β-D-Galf-(1→3)-α-D-Glcp-(1→
 catalysant spécifiquement l'une des liaisons suivantes entre sucres: la liaison osidique β-1,3 entre le carbone 1 d'un D-Gal*f* activé et le carbone 3 du Glc*p* présent à l'extrémité non-réductrice d'une chaîne saccharidique en formation ; la liaison osidique β-1,3 entre le carbone 1 d'un D-Glc*p* activé et le carbone 3 du Gal*f* présent à l'extrémité non-réductrice d'une chaîne saccharidique en formation ; la liaison osidique β-1,6 entre le carbone 1 d'un D-Gal*p* activé et le carbone 6 du Glc*p* présent à l'extrémité non-réductrice d'une chaîne saccharidique en formation ; et la liaison osidique α-1,3 entre les unités saccharidiques répétitives de l'EPS ci-dessus. L'invention concerne aussi tout ADN recombinant codant pour une enzyme recombinée selon l'invention, ainsi que toutes cellules comprenant, intégrées dans son génome ou par le moyen d'un plasmide réplicable, un ADN recombiné, et exprimant une enzyme recombinée fonctionnelle selon l'invention. Enfin l'invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

## Description

La présente invention se rapporte à de nouvelles fonctions enzymatiques de bactéries lactiques alimentaires, ainsi que de nouveaux enzymes et gènes, impliqués dans la biosynthèse d'exopolysaccharides.

### État de la technique

Il est connu que les bactéries lactiques sont susceptibles de produire dans leur milieu de culture deux classes de polysaccharides, à savoir les homopolysaccharides comme les dextranes ou les levanes qui sont constitués par l'assemblage répété d'un seul sucre, et les hétéropolysaccharides appelés communément exopolysaccharides ou EPS (EPS est l'abréviation du terme "exopolysaccharide") constitués par l'assemblage de plusieurs sucres différents formant une unité répétitive (Cerning J., Bactéries lactiques, Vol I, de Rossart H et Luquet F. M., Lorica, 309-329, 1994).

Une bactérie lactique alimentaire produisant un EPS peut conférer un caractère filant et/ou une texture lisse et crémeuse à un lait acidifié (Cerning *et al*., FEMS Microbiol., 87, 113-130, 19/90). Les EPS peuvent aussi présenter des activités biologiques particulièrement intéressantes pour la santé humaine ou animale, comme des activités anti-tumeurs ou probiotiques, par exemple (Oda M. *et al*., Agric. Biol. Chem., 47, 1623-1625, 1983; EP94870139.6)

Par ailleurs, l'industrie alimentaire est confrontée à une instabilité génétique de la biosynthèse des EPS dans les bactéries lactiques. Ceci se traduit généralement au cours d'une fermentation par la perte de la production d'EPS par tout ou partie des bactéries lactiques (voir "Cerning J." ci-dessus). Les produits fermentés industriels sont ainsi sujets à des variations dans leur contenu en EPS, ce qui n'est pas toujours acceptable. Pour remédier à ces problèmes, l'industrie recours actuellement à l'isolement et la caractérisation périodique de ses bactéries de manière à séparer celles qui ont perdu leur caractère originel.

On connaît des gènes de bactéries lactiques alimentaires impliqués dans la biosynthèse d'EPS.

WO92/02142 révèle ainsi l'existence du plasmide pHV67 qui produit dans *Lactococcus lactis* subsp. *lactis* (mésophile) une substance capable d'augmenter la viscosité d'un lait fermenté. La structure saccharidique de cette substance n'étant pas connue, les différentes fonctions des enzymes codées par pHV67 sont également inconnues.

US5066588 décrit deux plasmides provenant d'une souche de *Streptococcus cremoris* (mésophile) capable de conférer un caractère épaississant à un *Streptococcus lactis*. La structure saccharidique de cet épaississant n'étant pas connue, les différentes fonctions des enzymes codées par ces plasmides sont également inconnues.

Vescovo *et al*. ont mis en évidence un plasmide d'une souche *Lactobacillus casei* subsp. *casei* (mésophile) codant pour un phénotype Muc+, c'est à dire pour des fonctions liées à la production d'épaississants exocellulaires (Vescovo *et al*., Biotechnology Letters, Vol II, 709-712, 1989). La structure saccharidique de cet épaississant n'étant pas connue, les différentes fonctions des enzymes codées par ce plasmide sont également inconnues.

EP750043 (Société des Produits Nestlé) décrit un opéron de gènes de la souche *Streptococcus thermophilus* CNCM I-1590 impliqué dans la synthèse d'un EPS ayant la structure répétitive suivante.

→3)-β-D-Glcp(1→3)-α-D-GalpNAc(1→3)-β-D-Galp(1→
6
↑
1
α -D-Galp

Des travaux menés sur ces enzymes (résultats non-publiés) ont permis de montrer que la biosynthèse de cet EPS se fait de manière progressive avec, à chaque étape, l'addition d'une nouvelle unité de sucre qui vient s'attacher par sa fonction semi-acétalique à un hydroxyle alcoolique d'une autre unité de sucre, laquelle est à l'extrémité d'une chaîne de sucres liée à une amorce. Ces enzymes catalysent ou contrôlent ainsi spécifiquement les fonctions suivantes.
(1) La liaison sous forme d'un isomère α ou β entre le carbone 1 (qui porte la fonction réductrice aldéhyde) d'un D-Gal*p* activé et un phosphate d'une amorce lipophilique notamment du genre undécaprénol, ou protéique notamment du genre ACP (Acyl Carrier Protein), par exemple.
(2) La liaison osidique α-1,3 entre le carbone 1 d'un Gal*p*NAc activé (UDP-Gal*p*NAc) et le carbone 3 du D-Gal*p* lié à son amorce.
(3) La liaison osidique β-1,3 entre le carbone 1 d'un D-Glc*p* activé (UDP-D-Glc*p*) et le carbone 3 du sucre présent à l'extrémité non-réductrice de la chaîne β-D-Gal*p*(1→3)-α/β-D-Gal*p*-amorce.
(4) La liaison osidique α-1,6 entre le carbone 1 d'un D-Gal*p* activé et le carbone 6 du sucre présent à l'extrémité non-réductrice de la chaîne β-D-Glc*p*(1→3)β-D-Gal*p*(1→3)-α/β-D-Gal*p*-amorce.
(5) Le transport des unités saccharidiques répétitives de l'EPS décrit ci-dessus à l'extérieur de la cellule bactérienne.
(6) La liaison osidique β-1,3 entre les unités saccharidiques répétitives de l'EPS ci-dessus.
(7) La régulation du nombre d'unités saccharidiques dans l'EPS, et donc de son poids moléculaire final.

Van Kranenburg *et a*l. ont aussi décrit un opéron de gènes, présent sur un plasmide d'une souche *Lactococcus lactis*, impliqué dans la synthèse d'un EPS ayant la structure répétitive suivante (Mol. Microbiology, 24, 387-397, 1997).

→4)-β-D-Galp(1→4)-β-D-Glcp(1→4)-β-D-Glcp(1→
2 3
↑ ↑
1 PO4
α-L-Rhap ↑
1
α-D-Galp

Bien que les enzymes codant pour cet EPS soient maintenant connues, il reste encore à élucider la spécificité de chaque enzyme. Pour le moment on peut valablement supposer, au regard des homologies avec d'autres opérons impliqués dans la biosynthèse de polysaccharides, que la biosynthèse débute par la liaison d'un Glc*p* activé sur une amorce lipophilique ou protéique.

Les connaissances relatives à la biosynthèse des EPS dans les bactéries lactiques alimentaires sont encore néanmoins très fragmentaires. Cependant il est maintenant admis que, basée sur les structures d'EPS élucidées jusqu'à présent et par analogie avec la production de composés tels que les lipopolysaccharides (Whitfield *et al*., Adv. Microbiol. Physiol., 35, 136-246, 1993), la synthèse débute par l'activation de monomères de sucres (glucose, galactose...) en sucres nucléotidiques utilisés comme précurseurs par les glycosyltransférases. La première étape de la biosynthèse de l'unité répétitive est catalysée par une glycosyltransférase particulière qui reconnaît, d'une part, un précurseur de type sucre nucléotidique et, d'autre part, une amorce lipophilique, notamment du genre undécaprénol-phosphate, ou protéique notamment du genre ACP, par exemple. Les autres glycosyltransférases agissent ensuite séquentiellement en ajoutant un sucre spécifique sur l'unité répétitive en construction. Les unités répétitives complètes sont ensuite exportées vers le milieu extracellulaire avant d'être polymérisées.

A ce jour, il existe toujours un besoin de disposer de nouvelles enzymes impliquées dans la synthèse d'EPS, et notamment des enzymes catalysant des liaisons spécifiques entre des sucres. La présente invention vise à remplir ces besoins.

### Résumé de l'invention

A cet effet, la présente invention concerne toute enzyme recombinée, que l'on peut purifier et qui est susceptible d'être impliquée dans la biosynthèse de l'EPS ayant l'unité saccharidique répétitive 
β-D-Galp1
↓
6
→3)-β-D-Glcp-(1→3)-β-D-Galf-(1→3)-α-D-Glcp-(1→
 catalysant spécifiquement l'une des liaisons suivantes entre sucres:
- la liaison osidique β-1,3 entre le carbone 1 d'un D-Gal*f* activé et le carbone 3 du Glc*p* présent à l'extrémité non-réductrice d'une chaîne saccharidique en formation, notamment la chaîne α/β-D-Glc*p*-amorce ;
- la liaison osidique β-1,3 entre le carbone 1 d'un D-Glc*p* activé et le carbone 3 du Gal*f* présent à l'extrémité non-réductrice d'une chaîne saccharidique en formation, notamment la chaîne β-D-Gal*f*(1→3)-α/β-D-Glc*p*-amorce ;
- la liaison osidique β-1,6 entre le carbone 1 d'un D-Gal*p* activé et le carbone 6 du Glc*p* présent à l'extrémité non-réductrice d'une chaîne saccharidique en formation, notamment la chaîne β-D-Glc*p*(1→3)β-D-Gal*f*(1→3)-α/β-D-Glc*p*-amorce ; et,
- la liaison osidique α-1,3 entre les unités saccharidiques répétitives de l'EPS ci-dessus.

Un autre objet de l'invention concerne tout ADN recombiné codant pour une enzyme selon l'invention, et notamment tout vecteur d'ADN ou toute cellule renfermant un tel ADN recombiné, et exprimant une enzyme recombinée fonctionnelle.

Un autre objet de l'invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produites par la cellule hôte, pour la biosynthèse d'un EPS, (3) puis on cultive la cellule hôte transformée dans des conditions appropriées pour la production d'un EPS.

Enfin, un dernier objet de la présente invention concerne l'utilisation d'une enzyme recombinée selon l'invention, ou d'un ADN selon l'invention, pour la synthèse d'un EPS.

### Description de la figure

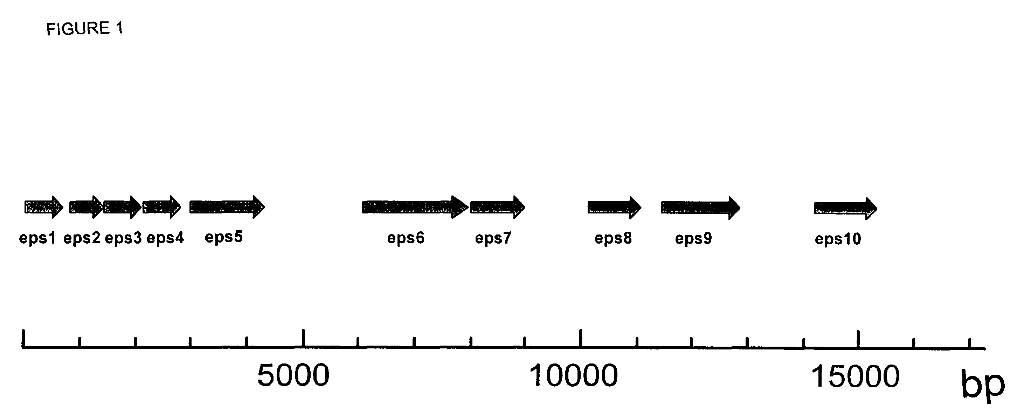

La figure 1 représente la carte physique de l'opéron impliqué dans la synthèse de l'EPS de la souche *S. thermophilus* CNCM I-1879. Les flèches horizontales indiquent la présence de cadres de lectures (ORF) potentiels. Les noms des gènes correspondants aux ORFs sont indiqués en dessous des flèches.

### Description détaillée de l'invention

Dans la suite de la description, les séquences en nucléotides des gènes ou amorces utilisés, ainsi que les séquences en acides aminés des enzymes recombinées, sont représentées dans la liste de séquences fournie ci-après, et sont nommées, pour des raisons de simplification, sous les sigles numérotés "SEQ ID NO:".

Le terme "EPS" désigne un polymère formé par la condensation d'un grand nombre de molécules de sucres (= oses) de types différents. Cet EPS est plus particulièrement constitué par l'assemblage répété d'une seule unité saccharidique, elle-même formée d'une chaîne principale de sucres dont les extrémités sont impliquées dans la polymérisation des unités. Cette chaîne principale peut comprendre en outre des ramifications de sucres qui ne sont pas impliquées dans la polymérisation des unités, lesdites ramifications étant greffées par des liaisons osidiques sur un ou plusieurs sucres de cette chaîne.

On représente une unité saccharidique en indiquant (1) les sigles usuels des sucres (Glc: glucose; Gal: galactose; Rha: rhamnose; etc.) ; (2) le type de chaque liaison osidique sous la forme d'une flèche entre les numéros de carbone de deux sucres adjacents (les numéros de carbone attribués sur le cycle d'un sucre sont ceux reconnus par la nomenclature internationale des sucres: IUPAC) ; (3) l'anomérie de chaque liaison osidique sous la forme du sigle α ou β, ledit sigle étant positionné à gauche d'un sucre pour indiquer l'anomérie de la liaison osidique présente à la droite de ce sucre; (4) la nature pyranose ou furanose de chaque sucre en adjoignant la lettre *p* ou *f* juste après le sigle de chaque sucre ; (5) et la série D ou L de chaque sucre, en positionnant la lettre à gauche du sucre concerné.

Dans une chaîne saccharidique en formation, la réaction directe entre une fonction semi-acétalique d'une nouvelle unité de sucre et un hydroxyle alcoolique du sucre présent à l'extrémité non-réductrice de la chaîne ne se fait pas spontanément, et c'est pourquoi le groupement semi-acétalique doit au préalable être activé, c'est-à-dire rendu plus réactif en vue de la formation de la liaison osidique. Cette activation est catalysée par des enzymes bactériennes formant un sucre nucléotidique, et plus particulièrement un uridine-diphosphate de D-Glc*p* (UDP-Glc*p*), D-Gal*p* (UDP-Gal*p*) ou D-Gal*f* (UDP-Gal*f*) dans le contexte de la présente invention. Pour initier la formation d'un chaîne saccharidique, un premier sucre activé doit d'abord être lié sur le phosphate d'une amorce lipophilique ou protéique. L'addition d'une nouvelle unité de sucre activé sur la chaîne se fera ensuite par sa fonction semi-acétalique au niveau d'un hydroxyle alcoolique du sucre présent à l'extrémité non-réductrice de cette chaîne, c'est-à-dire à l'extrémité opposée de celle où l'amorce est fixée.

Dans la suite de la description, on représente une chaîne saccharidique en formation, en indiquant les sigles usuels des sucres, les types de liaisons osidiques, la position de l'amorce sur la chaîne, la nature du cycle et de la série D ou L de chaque sucre. Enfin, du fait que l'on ne sait pas encore quel isomère est formé lorsque le premier sucre d'une chaîne est lié à une amorce, par commodité d'écriture, on utilise ainsi le sigle "α/β" afin d'indiquer que l'un des deux isomères est formé.

Au sens de la présente invention, on entend par "séquence homologue" toute séquence nucléotidique ou d'acides aminés, conduisant ou donnant un variant d'enzyme qui catalyse l'assemblage de sucres, ne différant des séquences selon l'invention que par la substitution, la délétion ou l'addition d'un petit nombre de bases nucléotidiques ou d'acides aminés, par exemple 1 à 150 paires de bases (pb) ou 1 à 50 acides aminés. Ces variants d'enzyme peuvent conserver la même spécificité d'assemblage de sucres que les enzymes dont ils dérivent. La substitution, la délétion ou l'addition d'un petit nombre d'acides aminés peut cependant aussi conduire à changer la spécificité enzymatique. Par exemple, les protéines codées par les gènes de l'invention *eps1* à *eps5*, bien qu'étant très proches des enzymes EpsA-D de la souche *S. thermophilus* Sfi6 (EP750043) et de l'enzyme EpsE de *S. salivarius*, présentent des spécificités enzymatiques différentes des protéines EpsA-D et EpsE (voir ci-après).

La sélection d'une séquence homologue est considérée comme évidente pour l'homme du métier, puisque l'on peut facilement créer des variants d'enzymes selon l'invention, en mettant en oeuvre des méthodes de mutagénèse classiques, et notamment en adaptant les méthodes décrites par Adams *et al*. (EP402450; Genencor), par Dunn *et al*. (Protein Engineering, 2, 283-291, 1988), par Greener *et al*. (Strategies, 7, 32-34, 1994) et/ou par Deng *et al*. (Anal. Biochem, 200, 81, 1992), par exemple.

Dans ce cadre, on considérera en particulier comme homologues deux séquences d'ADN qui, du fait de la dégénérescence du code génétique, codent pour un même polypeptide. De même, on peut aussi considérer comme homologue deux séquences d'ADN s'hybridant dans des conditions stringentes, c'est-à-dire s'hybridant toujours après une étape d'hybridation à 65°C pendant 15h dans un tampon d'hybridation (SSPE 1,5x: 0,225 M de NaCl, 0,0015 M d'EPTA, 0,015 M de NaH₂PO₄, pH7 ; 1% de SDS et 1% de lait déshydraté), suivie de 6 étapes successives de lavage à 65°C dans un tampon comprenant différentes dilutions de SSC 10x (1,5 M de NaCl, 0,15 M de citrate de sodium, pH7) et 0,1% SDS, respectivement pendant 2 fois 10 min avec du SSC 2x, pendant 2 fois 10 min avec du SSC 1x, et pendant 2 fois 5 min avec du SSC 0,1x.

On peut aussi considérer comme homologues les séquences d'ADN présentant plus de 50% d'identité avec les séquences selon l'invention, en particulier plus de 70%, l'identité étant déterminée par le rapport entre le nombre de bases nucléotidiques d'une séquence homologue qui sont identiques à celles d'une séquence selon l'invention, et le nombre total de bases nucléotidiques de ladite séquence selon l'invention, par exemple.

De même, on considérera comme homologues deux protéines fonctionnelles qui sont reconnues par un même anticorps, le rapport des valeurs d'intensité de reconnaissance des deux protéines par l'anticorps n'excédant pas 1000 dans un test ELISA, de préférence 100, par exemple. On considérera plus particulièrement comme homologues les séquences en acides aminés qui présentent plus de 70% d'identité avec les séquences selon l'invention, en particulier plus de 80% ou 90%. Dans ce dernier cas, l'identité est déterminée par le rapport entre le nombre d'acides aminés d'une séquence homologue qui sont identiques à celles d'une séquence selon l'invention, et le nombre total d'acides aminés de ladite séquence selon l'invention.

Pour isoler un ADN recombiné selon la présente invention, il est possible de constituer une banque de grands fragments d'ADN d'une bactérie lactique produisant un EPS dans une bactérie lactique ne produisant pas d'EPS, puis de sélectionner le ou les clone(s) produisant un EPS. Pour cela, on digère l'ADN génomique d'une bactérie lactique produisant un EPS par une enzyme de restriction qui est spécifique d'un site de restriction relativement rare (*Bam*HI, *Sal*I, *Pst*I) ou par une digestion partielle avec *Sau*3A, par exemple. On clone le produit de digestion dans un plasmide d'expression ou d'intégration qui accepte de grands fragments (plasmide pSA3, Dao *et al*., Appl. Environ. Microbiol., 49, 115-119, 1985), on introduit les plasmides recombinants dans la même espèce de bactérie lactique ne produisant pas d'EPS, on sélectionne au moins un clone transformé produisant un EPS, puis on identifie, on isole et on séquence classiquement le fragment d'ADN responsable de la production d'EPS.

Vu que les ADN recombinés selon la présente invention sont susceptibles d'être de grande taille, du fait qu'ils peuvent contenir un groupe de gènes impliqués dans la biosynthèse d'EPS, on peut préférer introduire les plasmides recombinants dans la même souche de bactérie lactique dont provienne les fragments, à la différence près que cette souche a perdu la capacité de produire des EPS suite à un traitement mutagénique (traitement U.V., chimique ou par transposon).

Une alternative à la méthode décrite ci-dessus peut aussi consister à constituer une banque plasmidique de fragments d'ADN d'une souche de bactérie lactique produisant un EPS, à transformer la même souche de bactérie lactique par les plasmides incapables de s'y répliquer, à sélectionner les transformants ayant intégré un plasmide dans leur génome par recombinaison homologue (sélection par une résistance à un antibiotique, par exemple), à sélectionner les transformants ne produisant plus d'EPS, puis à isoler et séquencer les fragments d'ADN génomique des transformants sélectionnés qui sont adjacents au plasmide intégré. Pour cela, on peut digérer le chromosome des transformants, le liguer, puis effectuer une PCR-inverse à l'aide de sondes spécifiques du plasmide intégré ou introduire le produit de ligation dans une souche dans laquelle le plasmide recircularisé est capable de se répliquer, par exemple.

Une autre alternative à la méthode de sélection décrite ci-dessus peut aussi consister à transformer des bactéries lactiques produisant un EPS par un plasmide comprenant un transposon, à soumettre les bactéries à des conditions dans lesquelles le transposon s'excise du vecteur et s'intègre au hasard dans le génome, à sélectionner les clones de bactéries ayant perdu la capacité de produire des EPS, à isoler les fragments d'ADN génomique desdits clones dans lesquels un transposon s'est intégré. Cette méthode est décrite plus en détail par Stingele *et al*., Dev. Biol. Stand., *In* Genetics of Streptococci, Enterococci and Lactococci, 85, 487-493, 1995 (ISSN:0301-5149).

Une autre alternative consiste à préparer des amorces nucléotidiques dérivées de gènes connus impliqués dans la biosynthèse d'un EPS, puis à réaliser une PCR sur l'ADN génomique d'une bactérie lactique produisant un EPS et pour laquelle on désire identifier les gènes impliqués dans la biosynthèse de cet EPS. Cette technique est cependant excessivement difficile à mettre en oeuvre, car le choix de la séquence nucléotidique des amorces sera déterminante dans l'isolement d'un gène. Puisque l'on ne connaît pas d'avance les homologies entre les gènes recherchés et les gènes connus, ce choix procède d'une démarche inventive, de par la nécessaire sélection opérée parmi une multiplicité d'amorces possibles.

Quelques critères peuvent cependant être retenus, tel que (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codées naturellement par 1 ou 2 codons, tel que la méthionine, le tryptophane, la glutamine ou l'acide glutamique, par exemple ; etc.

Il faut remarquer que les méthodes de sélection décrites brièvement ci-dessus peuvent être appliquées à toutes les bactéries lactiques connues, notamment aux bactéries lactiques mésophiles comme par exemple *Streptococcus cremoris*, *Streptococcus lactis*, *Lactobacillus casei* subsp. *casei* et *Lactobacillus sake*, et les bactéries lactiques thermophiles comme par exemple *Streptococcus thermophilus* et *Lactobacillus helveticus*. A cet effet, l'homme du métier dispose de techniques de transformation pour chaque espèce de bactérie lactique.

De plus, les méthodes de sélection décrites ci-dessus permettent le plus souvent d'isoler seulement une partie d'un gène ou d'un groupe de gènes impliqués dans la biosynthèse d'un EPS. Néanmoins, l'homme du métier peut facilement identifier la partie restante du gène ou du groupe de gènes en sélectionnant dans une banque génomique, à l'aide de sondes nucléotidiques basées sur un fragment isolé, un ou plusieurs clones renfermant la partie restante, par exemple.

On a pu ainsi caractériser une séquence d'ADN de 17,2 kb de la souche *Streptococcus thermophilus* déposée le 20 juin 1997 selon le traité de Budapest auprès de la Collection Nationale de Culture de Microorganisme (C.N.C.M.), 28 rue du Docteur Roux, 75724 Paris cedex 15, France, où elle a reçu le numéro de dépôt CNCM I-1897. Par ailleurs, cette souche Gram-positif présente au microscope un aspect de coques non flagellées formant des chaînettes. Cette souche ne fait pas de spores et elle est anaéorobe facultative. Toutes les restrictions concernant la disponibilité de cette souche seront irrévocablement levées lors de la délivrance d'un brevet correspondant à la présente demande ou une autre demande qui revendique le bénéfice de priorité sur cette demande.

Cette séquence de 17,2 kb comprend des gènes codant pour de nouvelles enzymes impliquées dans la synthèse de l'EPS ayant la structure répétée suivante:

β-D-Galp1
↓
6
→3)-β-D-Glcp-(1→3)-β-D-Galf-(1→3)-α-D-Glcp-(1→

Grâce aux connaissances acquises sur la biosynthèse des EPS décrits dans la littérature, notamment de l'EPS décrit dans EP750043 (résultats non-publiés), on a pu ainsi identifier les nouvelles fonctions enzymatiques impliquées dans la biosynthèse de l'EPS ci-dessus. Les nouvelles enzymes impliquées dans la synthèse de cet EPS peuvent ainsi catalyser spécifiquement l'une des nouvelles liaisons suivantes entre sucres:
- la liaison osidique β-1,3 entre le carbone 1 d'un D-Gal*f* activé et le carbone 3 du Glc*p* présent à l'extrémité non-réductrice d'une chaîne saccharidique en formation, notamment la chaîne α/β-D-Glc*p*-amorce ;
- la liaison osidique β-1,3 entre le carbone 1 d'un D-Glc*p* activé et le carbone 3 du Gal*f* présent à l'extrémité non-réductrice d'une chaîne saccharidique en formation, notamment la chaîne β-D-Gal*f*(1→3)-α/β-D-Glc*p*-amorce ;
- la liaison osidique β-1,6 entre le carbone 1 d'un D-Gal*p* activé et le carbone 6 du Glc*p* présent à l'extrémité non-réductrice d'une chaîne saccharidique en formation, notamment la chaîne β-D-Glc*p*(1→3)β-D-Gal*f*(1→3)-α/β-D-Glc*p*-amorce ; et,
- la liaison osidique α-1,3 entre tes unités saccharidiques répétitives de l'EPS ci-dessus.

Cinq de ces nouvelles enzymes recombinées présentent une faible homologie avec des enzymes connues. Ces nouvelles enzymes, identifiées à partir de la séquence d'ADN de 17,2 kb (*eps* 6-10), ont l'une des séquences en acides aminés choisie dans le groupe de séquences SEQ ID NO:7, SEQ ID NO:8, SEQ ID NO:9, SEQ ID NO:10, SEQ ID NO:11 et les séquences qui leur sont homologues.

Cette séquence d'ADN de 17,2 kb contient cependant aussi d'autres nouveaux gènes impliqués dans la synthèse de cet EPS (*eps1-5*), lesdits gènes codant néanmoins pour des protéines ayant de très fortes homologies avec les protéines EpsA-D de *S. thermophilus* Sfi6 (EP750043) et de la protéine EpsE de *S. salivarius* (GenBank: N°X94980). Ces hauts degrés d'homologie sont inattendus compte tenu du fait que la structure de l'EPS produit par la souche CNCM I-1897 est suffisamment différent de celui de la souche Sfi6, pour que l'on puisse s'attendre à ce que les enzymes de la souche CNCM I-1897 soient différentes de celles de la souche Sfi6.

Les protéines codées par les gènes *eps1-5*, bien que très proches des protéines EpsA-D et EpsE, présentent cependant des spécificités enzymatiques nouvelles les rendant particulièrement attractives pour la création de nouvelles liaisons entre sucres, ou pour faire varier la longueur de la chaîne d'EPS, ou même pour réguler différemment la production de l'EPS. Une de ces nouvelles enzymes est une glycosyltransférase ayant spécifiquement la séquence en acides aminés SEQ ID NO:6, les séquences homologues étant bien entendu exclues pour des raisons de nouveauté. Les autres nouvelles enzymes sont impliquées dans la régulation de la production de l'EPS et de la longueur de la chaîne, et ont spécifiquement l'une des séquences en acides aminés choisie dans le groupe de séquences SEQ ID NO:2, SEQ ID NO:3, SEQ ID NO:4 et SEQ ID NO:5, les séquences homologues étant bien entendu exclues pour des raisons de nouveauté.

La présente invention vise également tout ADN recombiné codant pour une enzyme selon l'invention, c'est à dire l'une des enzymes choisies dans le groupe de séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 et SEQ ID NO:11. En particulier, cet ADN peut comprendre au moins un gène ayant l'une des séquences nucléotidiques choisie parmi les séquences délimitées dans la séquence SEQ ID NO:1 par les nucléotides 1-687, 690-1418, 1429-2119, 2131-2870, 2929-4244, 5933-7978, 8003-8969, 10138-11086, 11438-12852, 14220-15315 et les séquences qui sont homologues aux séquences délimitées dans la séquence SEQ ID NO:1 par les nucléotides 5933-7978, 8003-8969, 10138-11086, 11438-12852 et 14220-15315.

La présente invention vise aussi tout vecteur recombinant comprenant un ADN selon l'invention. Ce vecteur recombinant peut être tout fragment d'ADN, simple ou double brin, linéaire ou circulaire, d'expression ou d'intégration, et comprenant une séquence d'ADN selon l'invention, notamment tout ou partie de la séquence SEQ ID NO:1. Dans le cas où le procédé décrit dans EP564966 ne serait pas utilisé (voir ci-après), il faut veiller à ce que le vecteur exprime l'ADN selon l'invention par des séquences nucléotidiques adaptées (promoteur; site d'attachement du ribosome; codon préféré par l'organisme hôte, etc.), et le cas échéant à ce qu'il comprenne une ou plusieurs origines de réplication cellulaire, notamment d'*Escherichia coli* et/ou d'un *Streptococcus*, par exemple.

Ainsi, pour opérer la biosynthèse d'un EPS, on peut intégrer tout ou partie de la séquence SEQ ID NO:1 comprenant au moins un des gènes précités dans une cellule hôte au moyen du procédé décrit dans EP564966, ledit procédé étant incorporé par référence dans l'enseignement de la présente invention. En résumé, ce procédé permet de pouvoir (1) transformer la cellule hôte avec un plasmide donneur qui ne s'y réplique pas, ledit plasmide comprenant ledit fragment placé fonctionnellement (le cadre de lecture est conservé) dans une partie d'un opéron issu de la cellule hôte; (2) identifier les transformants ayant intégré la totalité du plasmide; (3) sélectionner des transformants ayant uniquement intégré dans le chromosome le fragment selon l'invention, les autres séquences du plasmide s'étant excisées du chromosome; (4) et cultiver les transformants sélectionnés dans des conditions appropriées pour la production d'un EPS.

On peut noter que ce procédé permet de ne pas utiliser des séquences promotrice et d'activation traductionnelle fonctionnelles. De plus, les conditions de culture appropriées pour la production d'EPS sont à la portée de l'homme du métier, qui peut utiliser des milieux de culture standards, et choisir le pH, la température et l'agitation du milieu optimum selon la souche utilisée.

On peut aussi choisir de cloner tout ou partie de la séquence SEQ ID NO:1 comprenant au moins un des gènes précités dans un plasmide d'expression autoréplicatif en aval de séquences promotrice et d'activation traductionnelle fonctionnelles, et le cas échéant en amont d'un terminateur, puis de transformer une cellule hôte par le plasmide recombinant.

On peut aussi utiliser les enzymes recombinées pour modifier ou synthétiser *in-vitro* un oligosaccharide ou un polysaccharide comme un EPS, par exemple. Pour cela, il est préférable de purifier au moins une de ces enzymes, en surexprimant classiquement leur gène dans une cellule, et en les isolant classiquement, par précipitation et/ou chromatographie (du milieu de culture et/ou de la partie membranaire des cellules), par exemple.

Un autre objet de la présente invention concerne une cellule comprenant, intégré dans son génome ou par le moyen d'un plasmide réplicable, un ADN recombiné selon l'invention, et exprimant une enzyme recombinée fonctionnelle selon l'invention. Cette cellule peut faire partie du groupe des moisissures, des levures, des bactéries et des plantes, par exemple. De préférence, les levures appartiennent aux genres *Saccharomyces*, *Kluyveromyces*, *Hansenula* et *Pichia* ; les bactéries sont Gram négative ou positive appartenant au genre *Escherichia*, *Bacillus*, *Lactobacillus*, *Lactococcus*, *Streptococcus* et *Staphylococcus* ; les cellules de plantes appartiennent aux légumineuses, et aux espèces céréalières et ligneuses ; tandis que les moisissures sont les cellules traditionnellement utilisées pour préparer un koji comme les *Aspergillu*s, *Rhizopus* et/ou *Mucor*.

Un autre objet de la présente invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte ; (2) on transforme une cellule hôte par ledit vecteur, ladite cellule hôte utilisant les enzymes codées par le vecteur, le cas échéant en combinaison avec d'autres enzymes produites par la cellule hôte si le vecteur n'apporte pas tous les enzymes nécessaires à la biosynthèse d'un EPS, pour la biosynthèse d'un EPS ; (3) puis on cultive la cellule hôte transformée dans des conditions appropriées pour la production d'un EPS.

Si l'on ne met pas en oeuvre le procédé décrit dans EP564966, le vecteur doit alors comprendre en outre au moins une séquence promotrice fonctionnelle et au moins une séquence d'activation traductionnelle fonctionnelle, permettant l'expression des gènes codant pour au moins l'une des enzymes selon l'invention.

La présente invention est décrite plus en détail ci-après à l'aide du complément de description qui va suivre, qui se réfère à des exemples d'obtention de fragments d'ADN, de plasmides recombinants et de bactéries transformées selon l'invention. Ces exemples sont précédés d'une description des milieux de culture. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook *et al*. (Molecular Cloning, A Laboratory Manual, Cold Spring Harbor Laboratory Press, U.S.A., 1989). Les pourcentages sont donnés en poids/poids, sauf indication contraire.

### Milieux: (rajouter 1,5% de Bacto-agar pour un milieu solide)

- M17 (Difco,USA): tryptone 0,5%, soytone 0,5%, viande hydrolysée 0,5%, extrait de levure 0,25%, acide ascorbique 0,05%, sulphate de magnésium 0,025%, disodium-beta-glycérophosphate 1,9% et de l'eau.
- LM17: milieu M17 comprenant 1% de lactose.
- GM17: milieu M17 comprenant 1% de glucose.
- MSK: lait écrémé (poudre reconstituée à 10%) comprenant 0,1% d'extrait de levure.
- HJL: tryptone 3%, extrait de boeuf 0,2%, extrait de levure 1%, lactose 1% et KH₂PO₄ pH 6,5 0,5%.
- Rouge de Ruthénium: extrait de levure 0,5%, lait en poudre écrémé 10%, sucrose 1%, agar 1,5% et 0,08g/l de rouge de ruthénium (voir FR2632968).

### Exemple I clonage de l'operon de S. thermophilus CNCM I-1897 impliqué dans la synthèse d'un EPS

I.1. Sélection d'une souche *S. thermoph**ilus* productrice d'EPS: on cultive les souches de bactéries lactiques de la collection Nestlé dans un milieu liquide HJL et on en étale des dilutions sur un milieu solide Rouge de Ruthénium. Les souches productrices d'EPS demeurent de couleur blanche car les EPS empêchent le colorant de teinter leur paroi cellulaire. Par contre, les souches non-productrices se colorent en rouge du fait de l'affinité du colorant pour le peptidoglycane de leur paroi cellulaire. On a ainsi sélectionné parmi les bactéries lactiques productrices d'EPS la souche *S. thermophilus* Sfi39, qui a reçu le numéro de dépôt CNCM I-1897 et que l'on désignera dans la suite des exemples par l'expression "souche Sfi39".
I.2. Structure répétée de l'EPS: la structure de l'EPS produit par la souche Sfi39 a déjà été présentée dans la demande de brevet européen n°97111381.6, dont le contenu technique est incorporé par référence dans la description.
I.3. Clonage:
   L'approche tentée afin d'isoler les gènes *eps* de la souche Sfi39 est basée sur la comparaison de gènes *eps* connus (ceux de *S. thermophilus*) et de leurs homologues (gènes *cap*, *cps*, *rfb*) d'espèces variées, et même d'espèces non-apparentées phylogéniquement, comme des espèces Gram-négatif. Ces comparaisons permettent ainsi de définir des régions conservées entre les espèces, et d'utiliser des amorces nucléotidiques dérivées de ces régions conservées afin d'amplifier par PCR une partie interne de l'opéron *eps* de la souche Sfi39. Cette approche s'est malheureusement heurtée à une impossibilité d'amplifier un gène *ep*s, quel que soit les amorces choisies. Les raisons ayant conduit à ces échecs peuvent être multiples, par exemple liées à une faible homologie entre les gènes *eps* de la souche Sfi39 et ceux des régions conservées, ou à des conditions de PCR inadéquates, etc.
   Face à ces échecs, on a décidé de sélectionner, puis d'utiliser dans une PCR, des amorces nucléotidiques particulièrement dégénérées. Le choix de ces amorces spécifiques a en fait été déterminant pour l'amplification d'un gène *eps* de la souche Sfi39, et a été motivé par les considérations suivantes: (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codés naturellement par 1 ou 2 codons, tel que la méthionine, le tryptophane, la glutamine ou l'acide glutamique, par exemple; etc.
   Parmi les amorces testés, celles ayant les séquences nucléotidiques SEQ ID NO:12 et SEQ ID NO:13, ont permis d'amplifier un fragment de gène *eps* dans les conditions de PCR suivantes: successivement 2 min à 95°C, 1 min à 42°C, 20 s à 72°C, puis 35 fois de suite le cycle de 30 s à 94°C, 1 min à 42°C et 20 s à 72°C. Pour cela, l'ADN génomique de la souche Sfi39 a été au préalable isolée selon la technique de Slos *et al*. (Appl. Environ. Microbiol., 57, 1333-1339, 1991). On utilise environ 100 ng d'ADN génomique et la Taq polymérase.
   Un fragment de PCR de 143 pb a ainsi pu être isolé, puis cloné dans le plasmide linéarisé pGEMT (Promega, USA). Le séquencage de ce fragment par la méthode des didéoxynucléotides (kit f-mol® DNA Sequencing System, Promega) indique une séquence correspondant aux nucléotides 4006 à 4149 de la séquence SEQ ID NO:1.
   Ce fragment de PCR a été utilisé pour cribler une banque λ-ZAP Express (Stratagene, USA) renfermant des fragments d'ADN de la souche Sfi39. Pour cela, selon les recommandations du fournisseur on digère partiellement une préparation d'ADN dudit mutant par *Sau*3A, on sépare les fragments par une électrophorèse sur gel d'agarose, on coupe du gel les bandes correspondants à des fragments de 5 à 12kb, on élue l'ADN, puis on le ligue au vecteur λ-ZAP Express préalablement digéré par *Bam*HI. On encapside *in-vitro* le produit de ligation à l'aide du système GigagoldIII (Stratagene), on mélange ensuite les phages avec des *Escherichia coli* XL1Blue (Stratagene) selon les recommandations du fournisseur, puis on étale le mélange sur boîte de Pétri. On analyse ensuite les plaques recombinantes par hybridation de leur ADN transféré sur une membrane Hybond-N (Amersham Life Sciences, UK) avec le fragment de PCR préalablement rendu radioactif (kit Random Primed DNA Labeling, Boehringer-Manheim).
   Parmi les nombreuses plaques recombinantes, on a pu sélectionner par hybridation plusieurs plaques positives, desquelles on a ensuite isolé les vecteurs λ-ZAP Express, puis excisé les vecteurs pBK-PCMV (pCMV) renfermant l'insert génomique (voir les recommandations du fournisseur Stratagene). On a ensuite séquencé les inserts génomiques de ces vecteurs pCMV (kit f-mol® DNA Sequencing System). En recoupant les séquences nucléotidiques des différents inserts génomiques, on a pu ainsi caractériser une séquence de 17,2 kb correspondant à l'opéron de la souche Sfi39 impliqué dans la synthèse d'un EPS (voir la figure 1). La séquence nucléotidique de ce fragment de 17,2 kb est représentée dans la liste de séquence ci-après.
I.4. Analyse de la séquence SEQ ID NO:1: la séquence SEQ ID NO:1 présente l'opéron *eps* de la souche Sfi39. Cette séquence comprend 10 ORFs complets, dans la même orientation, que l'on appelle *eps1*, *eps2*, *eps3*, *eps4*, *eps5*, *eps6*, *eps7*, *eps8*, *eps9* et *eps10* (voir figure 1). La comparaison des séquences en acides aminés codées par les ORFs avec celles de protéines présentes dans la banque de donnée Swiss-Prot, à l'aide des logiciels FASTA, PEPPLOT et PILEUP de GCG-softwear, Wisconsin, USA, permet de déduire la fonction de ces protéines. Les résultats sont présentés ci-après.

Les ORFs *eps1* (nucléotides 1-687), *eps2* (nucléotides 690-1418), *eps3* (nucléotides 1429-2119), *eps4* (nucléotides 2131-2870) et *eps5* (nucléotides 2929-4244) ont plus de 80% d'identité avec les gènes *epsA*, *epsB*, *epsC* et *epsD* de la souche *S. thermophilus* Sfi6 décrits dans EP750043 (Genbank, n°U40830), et avec le gène *epsE* de *S. salvarius* (GenBank, n°X94980). Ces ORFs codent respectivement pour des protéines ayant plus de 90% d'identité avec les protéines EpsA, EpsB, EpsC, EpsD et EpsE de *S. thermophilus* et *S. salivarius*. Ces hauts degrés d'identité sont inattendus compte tenu du fait que la structure de l'EPS produit par la souche Sfi39 est suffisamment différent de celui de la souche Sfi6, pour que l'on puisse supposer que les enzymes de la souche Sfi39 soient différentes de celles de la souche Sfi6. Les protéines codées par les gènes *eps1-5*, bien que très proches des protéines EpsA-D et EpsE, présentent cependant des spécificités enzymatiques nouvelles (voir ci-dessus) les rendant particulièrement attractives pour la création de nouvelles liaisons entre sucres (*eps5*), ou pour faire varier la longueur de la chaîne d'EPS ou même pour réguler différemment la production de l'EPS (*eps1-4*). Les ORF *eps1*, *eps2*, *eps3*, *eps4* et *eps5* codent respectivement pour des protéines ayant les séquences en acides aminés SEQ ID NO:2, SEQ ID NO:3, SEQ ID NO:4, SEQ ID NO:5 et SEQ ID NO:6.

L'ORF *eps6* (nucléotides 5933-7978) code pour une protéine (SEQ ID NO:7) ayant environ 20% d'identité avec la protéine RfbC de *Klebsiella pneumaniae* (Genbank, n°L41518). Cette homologie confirme son rôle dans la biosynthèse de l'EPS.

L'ORF *eps7* (nucléotides 8003-8969) code pour une protéine (SEQ ID NO:8) ayant environ 33% d'identité avec la protéine EpsI de *S. thermophilus* Sfi6. Cette homologie indique que la protéine est de toute évidence une betaglycosyltransférase.

L'ORF *eps8* (nucléotides 10138-11086) code pour une protéine (SEQ ID NO:9) ayant environ 30% d'identité avec la protéine EpsI de *S. thermophilus* Sfi6. Cette homologie indique que la protéine est de toute évidence une betaglycosyltransférase.

L'ORF *eps9* (nucléotides 11438-12852) code pour une protéine (SEQ ID NO:10) ayant environ 40% d'identité avec la protéine EpsK de *Lactococcus lactis* (GenBank n°U93364). Cette homologie indique que la protéine est probablement le transporteur de l'unité répétitive dans le milieu extérieur.

L'ORF *eps10* (nucléotides 14220-15315) code pour une protéine (SEQ ID NO:11) ayant environ 62% d'identité avec la protéine Galf d'*Escherichia coli* (GenBank n°U03041). Cette homologie confirme son rôle dans la biosynthèse de l'EPS, en catalysant la conversion de la forme pyranose à la forme furanose d'un β-D-Gal.

En conclusion, les inserts génomiques isolés des vecteurs pCMV couvrent une région génomique de la souche *S. thermophilus* Sfi39 qui est manifestement impliquée dans la biosynthèse de l'EPS.

### Exemple II: inactivation des gènes eps

On inactive par recombinaison homologue les gène *eps1-10* de l'opéron pour confirmer leur importance dans la biosynthèse de l'EPS. Pour cela, on amplifie par PCR un fragment de chaque ORF provenant d'un des vecteurs pCMV décrits à l'exemple I ci-dessus, on ligue le produit de PCR dans le plasmide thermo-sensible pSA3 préalablement digéré, on transforme la souche *E. coli* XL1-blue par le produit de ligation, on sélectionne des transformants, on isole un plasmide recombinant, puis on transforme par électroporation la souche *S. thermophilus* Sfi39 avec le plasmide recombinant au moyen d'une méthode adaptée de celle décrite par Slos *et al*. (Appl. Environ. Microbiol., 57, 1333-1339, 1991). On resuspend les cellules soumises à une décharge de 2,1kV, 25µF et 400Ω dans 1 ml de milieu HJL que l'on incube 4h à 37°C (température permissive), on étale les cellules sur un milieu solide LM17 supplémenté de 2,5µg/ml d'erythromycine que l'on incube 16 h à 37°C, puis on sélectionne les colonies transformées qui survivent. On incube ensuite les colonies sélectionnées dans 2 ml de milieu HJL supplémenté de 2,5 µg/ml d'erythromycin jusqu'à ce que la densité optique à 600 nm (DO₆₀₀) de la culture atteigne 0,2, on soumet la culture à 45°C jusqu'à ce que la DO₆₀₀ atteigne 1,0 (le plasmide ne se réplique plus), puis on étale des dilutions de la culture sur un milieu LM17 solide supplémenté de 2,5 µg/ml d'erythromycine que l'on incube 12 h à 45°C.

Les colonies qui survivent ont intégré dans un des 10 gènes *ep*s le plasmide pSA3 recombinant. Ceci peut être vérifié par Southern-Blot d'une préparation d'ADN génomique des colonies survivantes digérée par *Eco*RI (coupe une seule fois dans pSA3), et hybridation du filtre de Southern-Blot avec le produit de PCR rendu radioactif. Les colonies ayant intégré le plasmide pSA3 présentent des bandes différentes que celles obtenues dans les mêmes conditions avec la souche sauvage. De plus, les colonies ayant intégré dans un des 10 gènes *eps* le plasmide pSA3 recombinant présentent un phénotype EPS(-) sur un milieu solide Rouge de Ruthénium, et ont perdu leur caractère filant dans un lait MSK.

### Exemple III Biosynthèse d'un EPS

On prépare un plasmide à partir du plasmide pSA3 (Dao *et al*., Appl. Environ. Microbiol., 49, 115-119, 1985), en ajoutant un fragment d'ADN de la souche Sfi39 contenant la séquence de 17,2 kb décrite à l'exemple I (voir la liste de séquence), et en ajoutant les parties régulatrices de l'opéron *eps* de la souche *S. thermophilus* Sfi6 (EP750043), de sorte que l'expression (la transcription et la traduction) des gènes *eps* de la souche Sfi39 puisse être correctement effectuée dans *S. thermophilus*. On transforme ensuite par électroporation, avec ce plasmide, la souche *S. thermophilus* CNCM I-1422 qui a été déposée le 18 mai 1994 selon le traité de Budapest. N'importe qu'elle autre souche filante de *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal=2:2.

### Exemple IV Biosynthèse d'un EPS

On prépare un plasmide à partir du plasmide pSA3 en ajoutant un fragment d'ADN de la souche Sfi39 contenant la séquence de 17,2 kb décrite à l'exemple I (voir la liste de séquence), et en ajoutant les parties régulatrices de l'opéron *eps* de la souche *S. thermophilus* Sfi6 (EP750043), de sorte que l'expression (la transcription et la traduction) des gènes *eps* de la souche Sfi39 puisse être effectuée dans *S. thermophilus*. On transforme ensuite par électroporation, avec ce plasmide, la souche *S. thermophilus* CNCM I-1351 qui a été déposée le 5 août 1993 selon le traité de Budapest. N'importe qu'elle autre souche filante de *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal:Rha=1:3:2

### Exemple V Biosynthèse d'un EPS

On isole de l'ADN génomique de la souche CNCM I-1897 par le méthode de Slos *et a*l. (Appl. Environ. Microbiol., 57, 1333-1339, 1991). On digère la préparation d'ADN par *Pst*I et *Bam*HI, on sépare les fragments d'ADN par électrophorèse sur gel d'agarose 0,7%, on élue les fragments supérieurs à 20 kb, on ligue l'ADN extrait au vecteur pJIM2279 (obtenu de P. Renault, INRA, Jouy-en-Josas, Paris, France) préalablement digéré par *Pst*I et *Bam*HI puis déphosphorylé. On transforme la souche *Lactococcus lactis* MG1363 (J. Bacteriol., 154, 1-9, 1983), cultivée sur milieu GM17 à 30°C, par la méthode de De Vos *et al*. (Gene, 85, 169-176, 1989). On sélectionne les clones transformés par hybridation du DNA génomique des clones avec l'une des sondes dérivées de la séquence SEQ ID NO:1. Parmi les transformants, quelques clones positifs sont sélectionnés.

### Exemple VI Préparation de gènes fonctionnels homologues aux gènes eps6, eps7, eps8, esp9 et eps10

On prépare des dérivés fonctionnels des gènes *eps6*, *eps7*, *eps8*, *esp9* et *eps10* en mettant en oeuvre une méthode adaptée de celle décrite par Adams *et al*. (EP402450; Genencor). Pour cela, on isole chacun des gènes *eps* par PCR à partir de l'ADN génomique de la souche Sfi39. Les amorces utilisées sont choisies à partir de la séquence SEQ ID NO:1, de sorte à amplifier uniquement chaque gène. On clone chaque produit de PCR dans le vecteur pFG1 dérivé du vecteur pSA3 (Dao *et al*., Appl. Environ. Microbiol., 49, 115-119, 1985) qui contient, en plus du gène de sélection erythromycine, le gène de sélection chloramphenicol et le promoteur régulant la transcription et la traduction de l'opéron *ep*s de la souche Sfi6 (EP750043). On clone chaque produit de PCR fonctionnellement en aval de ce promoteur de sorte que le gène *eps* puisse être normallement exprimé dans *S. thermophilus*. On soumet enfin chaque vecteur à une mutagenèse chimique *in*-*vitro* avec de l'hydroxylamine, comme décrit par Adams *et al*..

On transforme la souche *E. coli* XL1-blue par les plasmides pFG1 traités avec l'agent mutagène, on sélectionne des transformants, on isole un plasmide recombinant, puis on incorpore le plasmide recombinant par électroporation dans un mutant de la souche *S. thermophilus* Sfi39 dont le gène *eps* qui est aussi porté par le plasmide pFG1 a été au préalable inactivé comme décrit à l'exemple II. La transformation est réalisée par une méthode adaptée de celle décrite par Slos *et al*. (Appl. Environ. Microbiol., 57, 1333-1339, 1991), au cours de laquelle, on soumet les cellules à une décharge de 2,1 kV, 25 µF et 400 Ω, on les resuspend dans 1 ml de milieu HJL que l'on incube 4 h à 37°C, on étale les cellules sur un milieu solide LM17 supplémenté de 2,5 µg/ml d'erythromycine et de 3 µg/ml de chloramphenicol que l'on incube 16 h à 37°C, puis on sélectionne les colonies transformées qui survivent.

Pour sélectionner des dérivés fonctionnels des gènes *eps6*, *eps7*, *eps8*, *esp9* et *eps10*, on cultive les souches de bactéries lactiques transformées dans un milieu liquide HJL, on en étale des dilutions sur un milieu solide Rouge de Ruthénium, on sélectionne les souches productrices d'EPS qui demeurent de couleur blanche, et on compare les propriétés rhéologiques des EPS produits par chaque clone à celle de l'EPS produit par la souche Sfi39. En observant notamment la viscosité et la force normale des EPS de chaque clone, on a pu ainsi identifier certains clones produisant un EPS ayant une propriété différente. L'analyse des gène *eps* portés sur les plasmide pFG1 des clones sélectionnés montre que ceux-ci ne différent du gène *eps* original que par la substitution, la délétion ou l'addition d'un petit nombre de bases nucléotidiques conduisant à des modifications de la séquence en acides aminés de la glycosyltransférase.

### Exemple VII Synthèse de polysaccharides avec les glycosyltransférases Eps6, Eps7, Eps8, Eps9 et Eps10

On isole chacun des gènes *eps* par PCR à partir de l'ADN génomique de la souche Sfi39. Les amorces utilisées sont choisies à partir de la séquence SEQ ID NO:1, de sorte à amplifier uniquement chaque gène. On clone chaque produit de PCR dans un vecteur d'expression pQE60 (Qiagen, US). Les vecteurs ainsi traités sont ensuite introduits dans la souche *E. coli* DH5α (Stratagen, US) qui ne possêde pas naturellement d'activité glycosyltransférase. On cultive les souches DH5α transformées dans du milieu LB contenant de l'ampicilline jusqu'à une DO₆₀₀ de 0,8, on ajoute au milieu de culture 1 mM d'IPTG, on incube les cellules pendant 2 h, on centrifuge les cellules à 6000 g pendant 10 min à 4°C, on les lave dans le tampon A (50 mM de Tris-acétate pH8, 1mM de DTT, 1 mM d'EDTA et 10% de glycérol), on les centrifuge à nouveau, on resuspend les cellules dans le tampon A contenant en outre 1 mM de PMSF (fluorure de phénylméthane sulfonyle) et on soumet la suspension à 15000 psi. On élimine les cellules entières par centrifugation à 6000 g pendant 15 min à 4°C, on récupère les membranes cellulaires du surnageant par ultracentrifugation à 100000 g pendant 1 h, et resuspend l'aliquot dans le tampon A. Ce protocole permet ainsi généralement d'obtenir environ 10 mg/ml de protéines.

Parallèlement, on cultive la souche Sfi39 dans un milieu contenant du D-Glc*p*, D-Gal*p* et D-Gal*f*, on soumet les cellules de cette culture à 20000 psi, on extrait des membranes les chaînes saccharidiques en formation (liées à une amorce) en traitant les débris cellulaires avec du chloroforme/méthanol (2:1), en évaporant l'extrait. A titre de témoin dans les essais qui vont suivre, on procède de la même manière avec la souche Sfi39 cultivée en présence de sucres radioactifs.

On détermine ensuite la spécificité des glycosyltransférases présentes dans les membranes par un procédé similaire à celui décrit par Kolkman *et al*. (Mol. Microbiol., 26, 197-208, 1997). Pour cela, on fait réagir pendant 2 h, dans un volume réactionnel de 150 µl, 100 µl (environ 1 mg) de membranes, 50 mM de Tris-acétate pH8, 10 mM de MgCl₂, 1mM d'EDTA, 1 µl (environ 25 nCi) d'UDP^{[14C]}-Glc*p*, d'UDP^{[14C]}-Gal*p* ou d'UDP^{[14C]}-Gal*f* selon la glycosyltransférase considérée, et le dépôt contenant les chaînes saccharidiques en formation non-marquées isolées de la souche Sfi39 (voir le paragraphe ci-dessus). Pour arrêter la réaction, on ajoute au volume réactionnel 2 ml de chloroforme:méthanol (2:1), on agite vigoureusement le mélange, et on le laisse reposer pendant 30 min. On élimine les sucres nucléotidiques en extrayant la phase organique 3 fois de suite dans 0,4 mi de PSUP (pour 1 litre: 15 ml de chloroforme, 250 ml de méthanol, 1,83 g de KCl et 235 ml d'eau). La phase inférieure contenant toutes les chaînes saccharidiques liées à une amorce est ensuite séchée sous vide, resuspendue dans 200 µl de 1-butanol et séparée dans deux tubes.

Dans la première série de tubes, on détache les oligosaccharides de leur amorce en effectuant une hydrolyse douce dans 50 mM de TFA (ac. trifluoroacétique) à 90°C pendant 20 min.

Dans la deuxième série de tubes, on soumet les oligosaccharides à une hydrolyse poussée dans une solution de 4M TFA pendant 1 h à 125°C.

On sèche ensuite les hydrolysats et on les resuspend dans 5 ml de sucres transporteurs dans 40% d'isopropanol (5 mg/ml de chaque sucre suivant: Glc, Gal, GalN, maltose, maltotriose, maltotetraose). Les sucres hydrolysées de chaque série de tubes sont alors déposés sur une plaque HPTLC silicagel 60 (Merck) que l'on soumet à un éluant composé de chloroforme, d'acide acétique et d'eau (respectivement 6:7:1). On autoradiographie ensuite la plaque pendant 16 à 24 h avec un film Biomax MS (Kodak). On visualise les sucres transporteurs en diffusant sur la plaque une solution d'éthanol comprenant 5% d'H₂SO₄, et en chauffant la plaque à 100°C pendant 15 min. L'analyse des plaques permet ensuite l'identification précise des fonctions enzymatiques codées par les gènes *eps* de la souche Sfi39.

LISTE DE SEQUENCES

(1) INFORMATIONS GENERALES:
(i) DEPOSANT:
(A) NOM: SOCIETE DES PRODUITS NESTLE
(B) RUE: 55 avenue nestle
(C) VILLE: vevey
(D) ETAT OU PROVINCE: vaud
(E) PAYS: suisse
(F) CODE POSTAL: 1500
(ii) TITRE DE L' INVENTION: BACTERIES LACTIQUES PRODUISANT DES
EXOPOLYSACCHARIDES
(iii) NOMBRE DE SEQUENCES: 13
(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:
(i) CARACTERISTIQUES DE LA SEQUENCE:
(A) LONGUEUR: 17266 paires de bases
(B) TYPE: nucléotide
(C) NOMBRE DE BRINS: double
(D) CONFIGURATION: linéaire
(ii) TYPE DE MOLECULE: ADN (génomique)
(ix) CARACTERISTIQUE:
(A) NOM/CLE: CDS
(B) EMPLACEMENT:1..687
(D) AUTRES INFORMATIONS:/product= "eps1"
(ix) CARACTERISTIQUE:
(A) NOM/CLE: CDS
(B) EMPLACEMENT:690..1418
(D) AUTRES INFORMATIONS:/product= "eps2"
(ix) CARACTERISTIQUE:
(A) NOM/CLE: CDS
(B) EMPLACEMENT:1429..2119
(D) AUTRES INFORMATIONS:/product= "eps3"
(ix) CARACTERISTIQUE:
(A) NOM/CLE: CDS
(B) EMPLACEMENT:2131..2870
(D) AUTRES INFORMATIONS:/product= "eps4"
(ix) CARACTERISTIQUE:
(A) NOM/CLE: CDS
(B) EMPLACEMENT:2929..4244
(D) AUTRES INFORMATIONS:/product= "eps5"
(ix) CARACTERISTIQUE:
(A) NOM/CLE: CDS
(B) EMPLACEMENT:5933..7978
(D) AUTRES INFORMATIONS:/product= "eps6"
(ix) CARACTERISTIQUE:
(A) NOM/CLE: CDS
(B) EMPLACEMENT:8003..8969
(D) AUTRES INFORMATIONS:/product= "eps7"
(ix) CARACTERISTIQUE:
(A) NOM/CLE: CDS
(B) EMPLACEMENT:10138..11086
(D) AUTRES INFORMATIONS:/product= "eps8"
(ix) CARACTERISTIQUE:
(A) NOM/CLE: CDS
(B) EMPLACEMENT:11438..12852
(D) AUTRES INFORMATIONS:/product= "eps9"
(ix) CARACTERISTIQUE:
(A) NOM/CLE: CDS
(B) EMPLACEMENT:14220..15315
(D) AUTRES INFORMATIONS:/product= "eps10"

(xi) DESCRIPTION DE LA SEQUENCE: SEQ ID NO: 1:

ACATGAATAC ACATAAGATT CTCTTGACGA CTACTCCACG TGATGCATAC GTTAAGATTC 60
CTGGTGGTGG AGCAGACCAG TATGATAAAT TAACCCACGC AGGTATTTAT GGCGTTGAAA 120
CATCTGAACA AACTCTGGAA GATCTATATG GTACTAAGAT TGATTACTAT GCCCGAATTA 180
ACTTCACATC TTTCCTTAAG TTGATTGACC AACTTGGTGG TGTGACAGTC CATAATGATC 240
AAGCTTTCAC AAGTCTTCAT GGGAAGTTTG ATTTCCCAGT TGGAGATATC CAAATGAATT 300
CAGAGCAAGC ACTTGGATTT GTTCGTGAAC GCTATAGTTT AGATGGCGGA GATAATGATC 360
GTGGTAAAAA CCAGGAAAAA GTCATTTCTG CGATTGTAAA CAAGTTGGCT TCTCTAAAGT 420
CTGTATCAAA CTTTACTTCA ATCGTTAATA ATCTCCAAGA CTCTGTTCAG ACAAATATTT 480
CTTTGGATAC CATTAATGCT TTGGCTAATA CACAACTTGA TTCAGGTTCT AAATTTACGG 540
TGACTTCTCA AGCAGTAACA GGTACAGGTT CAACCGGACA ATTGATCTCT TATGCGATGC 600
CAAATTCTAG TCTTTACATG ATGAAACTAG ATAATTCGAG TGTGGAAAGT GCCTCTCAAG 660
CTATCAAAAA ATTGATGGAG GAAAAATAAG TGATTGACGT TCACTCACAT ATTGTTTTTG 720
ATGTTGATGA TGGTCCTAAA ACTTTAGAAG AAAGTTTAGA CCTCATTGGT GAAAGTTACG 780
CCCAGGGGGT ACGTAAGATT GTTTCAACAT CCCATCGTCG TAAGGGAATG TTTGAGACTC 840
CAGAGGATAA AATTTTTGCC AACTTTTCTA AGGTAAAAGC AGAAGCAGAA GAACTTTATC 900
CAGACTTAAC TATTTATTAT GGAGGTGAAC TTTATTACAC CTCAGACATT GTGGAGAAAC 960
TTGAAAAGAA TCTCATTCCG CGCATGCACA ACACTCAATT TGCTTTGATT GAGTTTAGTG 1020
CTCGCACATC TTGGAAAGAA ATTCATAGTG GGCTTAGTAA TGTTTTGAGA GCGGGGGTAA 1080
CACCTATTGT TGCTCATATT GAGCGCTATG ATGCCCTCGA AGAAAATGCT GATCGTGTTA 1140
GAGAAATCAT CAATATGGGC TGCTATACTC AAGTCAATAG CTCACATGTC CTCAAACCAA 1200
AGCTCTTTGG AGATAAAGAT AAAGTAAGAA AGAAACGTGT TCGCTTTTTC TTGGAGAAAA 1260
ATTTGGTTCA TATGGTTGCT AGCGACATGC ATAATCTTGG GCCGAGACCA CCATTTATGA 1320
AAGATGCTTA TGAAATTGTT AAAAAGAACT ACGGCTCCAA ACGTGCTAAG AATCTTTTTA 1380
TTGAAAATCC CAAAACATTA CTAGAAAATC AATATTTATA GGAGATATTA TGAATCAAGA 1440
TAACACTAAA AGTGATGAAA TCGACGTACT AGCATTGCTA CATAAACTTT GGACGAAGAA 1500
GCTTTTGATT CTTTTCACAG CTTTTTATTT CGCTGCTTTC AGTTTCTTAG GTACTTATTT 1560
CTTTATCCAA CCAACATATA CATCAACAAC GCGTATCTAT GTGGTTAATC AGGCAACAGA 1620
TAATAAGAAT CTTTCTGCTG AAGCTTTGCA GGCCGGTACA TTTTTGACAA AAGACTACAA 1680
AGAAATTATT ACATCAAACG ATGTCTTGTC AGAAGTTATC AAAGATGAAA AATTGAATAT 1740
GACAGAAGCA GAACTTGCTA AAATGATTTC AGTTGATATT CCTACTGATA CTCGTCTTAT 1800
TTTAATTTCT GTTAATGCTA AAACTGGTCA AGATGCGCAA ACACTTGCCA ATAAGGTTCG 1860
TGAAGTTGCT TCAGAAAAAA TCAAGAAGGT GACAAAAGTT GAAGATGTCA CAACGCTCGA 1920
AGAAGCTAAA TTGCCAGAGT CACCATCTTC ACCAAATATC AAACTTAATG TGCTTCTTGG 1980
GGCAGTGCTT GGAGGATTCC TTGCAGTGAT TGGTGTATTG GTACGTGAAA TCCTAGATGA 2040
TCGTGTTCGC CGTCCAGAAG ATGTGGAAGA TGTGCTTGGA ATGACACTTC TTGGAATTAT 2100
TCCTGATACA GATAAAATTT AAGGAGAAGA AATGCCTTTA TTAAAGTTAG TTAAATCAAA 2160
AGTAGACTTT GCTAAAAAGA CGGAAGAGTA TTATAACGCT ATTCGCACAA ATATTCAATT 2220
TTCTGGTGCT CAGATTAAAG TGATTGCGAT TAGCTCTGTT GAAGCTGGTG AAGAAAAATC 2280
AACGACATCT GTTAACTTGG CAATTTCATT TGCTAGTGTT GGACTCCGAA CACTTCTGAT 2340
TGATGCTGAT ACGCGTAATT CTGTTTTGTC GGGTACATTT AAATCAAATG AGCCTTATAA 2400
AGGTCTTTCA AATTTCCTTT CGGGAAATGC CGATCTAAAT GAAACGATTT GCCAAACTGA 2460
TATTTCTGGT TTAGATGTTA TTGCATCTGG TCCTGTTGCA CCTAATCCAA CAAGTCTTTT 2520
GCAAAATGAT AATTTTAGAC ATTTGATGGA AGTTGCTCGT AGTCGTTATG ATTATGTCAT 2580
CATCGATACA CCACCAATTG GTCTGGTTAT TGATGCAGTT ATTATTGCCC ATCAGGCTGA 2640
TGCCAGTCTT TTGGTTACAG CAGCTGGAAA AATTAAACGT CGTTTCGTAA CTAAGGCCGT 2700
TGAACAATTG GAACAAAGTG GTTCTCAGTT CTTAGGGGTC GTCCTTAATA AAGTTGACAT 2760
GACAGTTGAT AAATATGGAT CGTATGGTTC TTACGGATCA TATGGCGAGT ATGGAAAAAA 2820
ATCTGACTAA AAAGAAGGTT ATTCAGGAGC ACATCGTCGT AGAAAAGGAT AGCATTAATG 2880
GGGATGATGC GGCTCCTTAT ACCTTAACAG ATTAAAAAGG GGTTTAGAGT GAGAGAAAAA 2940
CAAGAAATTC GTCGCATTGA AATTGGTATT ATACAGTTGG TTGTGGTTGT TTTCGCAGCC 3000
ATGGTAGCTA GTAAAATACC ATATACAGAG ATTACCCAAG GAAGCATTGT CCTTTTAGGT 3060
GTCGTACATG TAGTGTCTTA CTATATCAGT AGTTATTATG AAAATCTTAA GTATAGAGGC 3120
TACTTGGATG AACTCATTGC AACTGTCAAA TATTGTTTCA TATTTGCTCT AATTGTAACA 3180
TTTCTCTCGT TTTTTGCAGA TGGAAGTTTT TCAATCTCAC GTCGCGGACT TCTTTACGTC 3240
ACCCTGATTT CAGGTGTTCT CTTATACGTT ACAAATACTG TTCTTAAGTA TTTCCGCTCA 3300
TCTATTTATA CACGTCGTAA AAGTAACAAG AATATTCTCT TGATTTCTGA TCAAGCACGT 3360
CTAGAAAATG TTTTGTCTCG TATGAAAGAC AATATGGATG GTAGGATTAC AGCAGTTTGT 3420
GTCTTGGATA ATCCTTATTT TACCGATCCA TTTATCAAGA GTGTTAAACC TGAAAATTTG 3480
ATTGAATATG CGACACACTC AGTAGTAGAC CAAGTTTTGA TTAATCTGCC AAGTGGGCAG 3540
TATAAGATTT GGGATTATGC ATCACCTTTT GAGATCATGG GAATTCCAGT TTCTATTAAT 3600
TTGAATGCCC TTGAATTTAT GAATCAAGGT GAAAAACGTA TTCAACAATT GGGTCCTTTC 3660
AAAGTTGTTA CGTTTTCAAC GTATTTTTAT AGCTATGGAG ATATCTTGGC GAAACGTTTC 3720
CTCGATATCT GTGGAGCTCT AGTTGGTTTG TTGCTCTGTG GTATTGTAGG AATCTTCCTT 3780
TATCCTCTTA TTCGTAAAGA TGGAGGACCA GCCATTTTTG CTCAAGACCG TGTGGGAGAA 3840
AATGGACGTA TCTTCAAGTT TTATAAATTC CGTTCTATGT GTGTTGATGC GGAAGAAATC 3900
AAGAAGAATT TGATGGCACA GAATCAAATG TCTGGTGGTA TGTTTAAGAT GGACAATGAT 3960

CCACGTATTA CCAAAATTGG ACATTTCATT CGTAAAACGA GTCTTGATGA ACTTCCACAA 4020
TTTTGGAATG TTCTAAAAGG TGATATGAGC TTGGTTGGGA CACGTCCTCC AACAGTTGAT 4080
GAGTATGAAA AATATACACC TGAACAGAAA CGTCGTTTAA GTTTTAAACC TGGTATCACT 4140
GGTCTTTGGC AAGTAAGCGG TCGAAGTGAA ATTACTGATT TTGATGAAGT TGTAAAACTA 4200
GACGTTGCTT ATTTGGACGG ATGGACAATC TGGCGTGATA TCAAAATCTT ATTGAAAACG 4260
ATTAAAGTAG TAGTAATGAA GGATGGAGCA AAGTGATGGC TTTCACCATT TCTTTTAATG 4320
GTGATTAAAT GACAAAAACA GTTTATATCG TTGGTTCTAA GGGGATTCCA GCAAAATATG 4380
GTGGATTTGA GACCTTTGTT GAGAAGTTGA CAGAGTTCCA ACAAGACAAA GATATCCAAT 4440
ATTATGTAGC TTGTATGCGG GAAAACTCTG CAAAATCAGA CATTACAGCA GATGATTTTC 4500
AAACTTCGCA ACAGAACCCT AAAAAGAACT CCCTAACTGT CGTGGCTACT TTGTTTAGTC 4560
TAAACACTTT GAATAGTCCT ACAAGCTCAT AGTTTCCCTT TTATTAGTTG GTCCAAGGCC 4620
AATTCTACTT TTTGAAGCGA AAGAATATGG GGAGCGCCTT CCTTATTTAC TGATATGTAA 4680
ACCTGGAATT ACTGGTTATT GGACGACACA TGGTCGAAGT AAAGTTCTTT TTCCTCAACG 4740
AGCAGATTTA GAACTCTATT ATCTCCAATA TTACAGCACC AAGAACGACA TCAAGCTTCT 4800
AATACGTACA ATTTCACAAA TCATTAACGG ATTGGACGCT TACTAAAAAA TTAATGAAAA 4860
AATATTTGAA ATCATAACTC GAAAAATATT AAAAGATTAG ATATGTATCA TAAAACCCGA 4920
ATTGCTAGTT AATTTCATTG GAAAATAAAT AAGTCGTGCT ATCCTAATCT TAAACCACTA 4980
AGCATTAGAA AGCGCACGAC TTATATGACT TATAATAGCA CACTTCCAAA AGTTTTTGTT 5040
TATTTACTGA CAACCATTGA GACGCTTTAT CAAACGAGTG TTCCCCTTGA GGTTCAAAAC 5100
CGAAAGAACG TCCATCTCGC AACATCAGAT TGCTTAGTTA TCGCTTGTTA CCTATGGGGC 5160
GTACTGCATT TTAGTGAAAC GCTTAAAGCA AAGCACCAAT TGGCTCAAAG TTTATTTCCT 5220
AATTTCCTAG AATATTCTCA CTTTGTCCGC CGTTGTAATG CCCTCTTACC GAGTATCCAA 5280
GTCATTCGCC AAGCACTCGT ATTTAAAGAG GTTGAAGGAA TTAGTGTATC CATTATTGAC 5340
AGCTTCCCCA TTCCTTTGTG TCAGACTATT CGTAATTTCA GAAGCAAAGT TCTTGGAGAT 5400
TATGCAAATG TTGGCTACAA TGCTACAAAG GGATAGTACT TCTATGGATG TAAATGTCAT 5460
GCTTTAGTCA GTGAATCAGG CTATGTCATA GACTACACAA TTACTCCTGC TTCAATGGCT 5520
GATAGTTCAA TGACCGAGGA AGTGTTGAGT CAATTTGGGA CACCAACAGT CCTTGGAGAT 5580
ATGGGATATT TAGGTCAGTC ACTGCATGAT AGGCTGGAAT TAAAAGGAAT TGATCTAATT 5640
ACACCTGTCA GGAAGAACAT GAAGCAAAAG AAAATTCTTT TCCCTAATTT TTCAAAACGT 5700
AGAAAAGTGA TTGAGCGAGT TTTCTCTTTT TTGACAGATC TAGGAGCTGA GCGTTGTAAA 5760
AGTCGTTCGC CTCAAGGTTT TCAATTGAAA TTAGAGATGA TACTTTTAGC GTATTCTTTA 5820
CTGTTAAAAT CAGCTAAATC ACTGGAACCA GAGACTTTAA GATATTCTAT CGGGTATCAA 5880
GTCATGGCTA AATAATCAAC TAGCAATTCG GGTATCATAA AGGAGTGATT TAATGAAAAA 5940
AATTACAATA GCAGTTGCAA CGCATAAAAA ATATCAAATG CCAAAAGATA ATATTTATCT 6000
ACCAATTGAA TGTGGAGCAG TTTTAAGAAA AAATCACCTA GACTATATTG CTGATGATAG 6060
TGGAGATAAT ATTTCTGAAA AAAACAAGAA TTATTCAGAG TTAACAGCAC TATATTGGTT 6120
ATGGAAAAAT AATGATTCTG AGTATAAAGG ATTAGCACAC TATAGACGTC ATTTTTCAGA 6180
TAAAAAGGTG AGTATTTTTT CTACAGGTAA TTTTGATAAT ATACTTGATA GGACGGTACT 6240
TGAGGAACAT TTAGAGAAGT TTGATATTAT ACTTCCCAAG CTTCGACATT ACTATATTGA 6300
AACAATAGAA TCTCACTATA AACATACGCA TTTTGAAAAA GATTTAATAG CAACAGAAGA 6360
GATAATTAAA AAACTATATC CTGACTATCT TGATTTTTAT TATAGTGCAC TAAAAAGAAA 6420
ATCTGCTCAT ATGTTTAATA TGTTTATTAT GAAAGATAAA TATTTCAATA ATTATTGTGA 6480
ATGGTTATTT TCGATTCTTT TTGAATTAGA AAAAGTACTA GATATTTCAG AATATTCTCC 6540
CTTTCATGCA AGAGTATTTG GTCGTGTGAG TGAAATTTTA TTAGATGTAT GGATTTTCAA 6600
GAATAATTTA AATTTCACTG AAATTCCAGT TATGTTTATG GAAAAGCAAA ATTGGTGGGA 6660
TAAATCTAAA AGATTTATTT CTGCAAAGCT TTTCAACAAA AAATATTATT AGACTAGGAG 6720
AAATAAATTG CTTACAATCG GAATAATTTT AATAATTTTT ATGACTATTT TCGATTATTA 6780
TATACATAAA ACAGTATTTT CTCCGGTCTT TATGTTTAAT TCACTCTTTT TATTAATAAT 6840
ATCTCTTTCT TCTATGAGGT TATATAATTT GAGAGAATAT TCTATCAAAT CAATAGAAGT 6900
AATTGTGTTA GGGATGATAT TCTTCTCTTT AGGAGTATTT TGTACTCGTA TTGTTTCTCA 6960
TGAATTTTTA AAAAATCAAA ATAATGTTAT TAATTACGAT GATAATTTAA ATGTAAATTG 7020
GACTTTTTTA AAAATTCTTT TGATAGTTGT AACCACAGGA AACGTATTCT CTATTATTTT 7080
TTCCTTGAAA TTCCTTTTAG GAGGAGGTTC ATACTTAGAA TTGAGAAATA TGATATTGGG 7140
ATATAATGGA GCTGAACCAC TTATTACGAA TCCTCTCGTA AATATATTAA CAAGATATAT 7200
ATCGGGACCA GGGTTGACTG CTTTGATCCC ATTTTCTATT TTTTTTCTAA TTAGGAAAAA 7260
AAATATTAAA TTTTCCTTAA TTATTTTATT GAATCTTGTT TTGGCAACAT TATCATCCGG 7320
TGGTAGAATT TTACTTGTAT ATACTATTAT TCAATTGTTT ATAGGATTAT CTTATTCAAA 7380
AAAAAATATA CCAAAAAAAA TTAAGAAAGT AGTCATAATA TCAAGTATTA TATTTTTTAT 7440
ATCTATAATT GTCTTATCTA ACATCAGAAG TTCTAACAGT ATATATAGAG CGTTTTATGC 7500
ATACTTTTCG GGCCCTGTAG TTCTTTTATC TACTTGGATG ACTGATGTTG ATACTTATAA 7560
TATTCATTCA CATGGATTAG GATTTATTTA TCCCATCACA TATCTATTAA ATTCATTTTG 7620
TAATTTAATA GGAATTCCTA ACTCGATGTT GGCTAATGTT GTCATGTGGC AAGGAATGCC 7680
TCAGAATGAT TGGGTAGGCG TATTCCCTAA TCAATCGATG AACGCTTTTA GTACACTTTT 7740
CTATTTTTTC TATAAAGATT TTCGAGAATT TGGAGTTGCT TGCTTTTCTT TCCTTTTTGG 7800
GAGTATTTGT GGTTTTATTT ATTTTAAAGC GTTTATCGAA AGAAAAAGTA AATATCTAGT 7860
CTATTATTTA TTAGGGGTAC AGGCAATTAT CGGATCTTTT ATTATTTGGC AATTGGGGAG 7920
TACTGCGTTT TTCTTAAGTA TTGTATTTAC AATATTAAGT CTGAAATCAA AGAAATCATA 7980
ACTATAGAAA AGGAGATAAA ATATGACAAT CAGCATAGTA ATCCCAGTTT ATAATGTTCA 8040

AGATTACATA AAAAAGTGTC TAGATTCTAT ATTAAGCCAG ACATTTTCAG ATTTAGAAAT 8100
TATTCTTGTT GATGATGGTT CTACTGACTT GAGTGGAAGA ATTTGTGATT ATTATTCCGA 8160
AAATGATAAA CGTATTAAAG TAATCCACAC AGCAAATGGG GGACAGTCGG AAGCAAGGAA 8220
CGTTGGAATC AAAAATGCCA CATCAGAATG GATAACATTT ATTGATTCTG ATGACTACGT 8280
TTCTTCTGAT TATATAGAGT ATTTATATAA TTTGATTCAA GTACACAATG CAGATATTTC 8340
AATAGCTAGT TTTACCTATA TCACACCTAA AAAGATAATT AAGCACGGTA ACGGTGAAGT 8400
AGCTCTTATG GATGCAAAAA CTGCAATTCG GAGAATGTTA CTGAATGAAG GTTTCGATAT 8460
GGGAGTTTGG GGGAAAATGT ATCGAACGGA GTATTTTAAT AAATATAAAT TCGTTTCAGG 8520
AAAACTATTT GAAGATTCTT TAATTACATA CCAGATATTT TCAGAAGCTT CAACAATTGT 8580
TTTTGGAGCA AAGGATATTT ATTTTTATGT TAACAGGAAA AATTCTACTG TTAATGGTAC 8640
TTTTAATATA AAAAAGTTTG ATCTTATTGA AATGAATGAA GAAGCAAATA AGTTTATTAA 8700
ACATAAATTT CCAGATCTTT CATCTGAAGC ACATCGTCGA ATGATATGGG CATATTTTAG 8760
TACACTAAAT CAAGTTTTAT CATCAACTAA TGAACACGAT ATTGATTTAT ATGCGCCACA 8820
ATTAGTAGCT TATCTCCTTA AACAGGATAA ATTCATAAAA AGGAATACTT TTATTCCCAA 8880
AAGAGATAAG ATTGCATTTT TTATTTTAAA AAATTTTGGT TTAAAGACAT ATCGTAATGT 8940
TTGGAATTTA TATTTAAAAA TGACAAGATA AAAACAATAA TGAAAGATAA AAAATAATGA 9000
AAACTGCTAC AATTACTTTA CATTCAGCAC ATAATAATGG ATCTATCTTT TCTACAGTCA 9060
TTTGCTTTGC AGAGAAAGAT AATATCTATG GGATATGATA ACGAAATTAT AAATTATATT 9120
CCACTCAAAT TGCTCGTAGT TTTAAGAACC AAGTGCAAAA GATTATTAAG GTTAACAACG 9180
TTACGATTGT TGCTACGGCC GCAATGCCGA AGAAGCCGCT GAATACGTTG ATTGGGCTAG 9240
TTGTCGGCTT GCTATAGGAT TTGTTTATGC AGCGATTCGG ATGCTCACGG ATCGCCGCGT 9300
TCACGAAGCT GATTTCTTAA CTGATGAATT AGGATTGACT AGTTTAGGCT TGGTTAACCA 9360
TCAACATCAC CATTCAATGA AGAAACAGGC CTTGAATCTG AACGATGGCT ATCATACGCA 9420
TAACGATCAA ACATCTTCAC AAGCGATGAA ACGAGTTTAG GAGGGTGCTA GATGTTTAAA 9480
CGTAAAGAAA AGAATATAAC GACTACGGCA CCAATTAATC TCACCACGAT TAATGAACCC 9540
ATGACGGTCA TTACTGAGCA GATTAAAACA ATTCGAACCA ATATCAATTT TGCGGCTACT 9600
GACCATAAGC TACGAACTTT GATGGTGACT TCGGCCATGC TTGGCGAGGG TAAATCGACA 9660
GTAAGTGGTA ACTTAGCAGT GGAGTATGCC AAGGAAGGAA GGCAAGCAAG TCTTACTGGT 9720
CGATGCTGAC TTGCGACGAC CAACGATTCA TAAGACATTT GGCCTTAAAA ACCATAAGGG 9780
ATTAAGTTCA TGGTTAGCCA ATCAGATTGA CGATGTGAAT GATGCAATTC ATCCCGTCAT 9840
TGGCAATCTA TAATTGTATA AATAGTATGT ATACTTTATA ACAATGGAGT GTTTTAATGA 9900
ATCTTTTGTT TAGTCAATGT CACATTACAT TGAAAATTTA AAAAATGTAA CTTTTTTGCG 9960
TGTGAATAGC TATGTACAAT GATTTTCTGG GTGGCAGACT AATCAAGTAT AAAATAGCTT 10020
ATACTAGTTG ACAACATCCC GTGATAATTA TTAACTTATC AAGTACAGGC CAAAATACTG 10080
GAGCTTAACA GGAACTGTTA GAATATGATT TTATATAATT AGGAGTAGAA TAAAGAGATG 10140
AATCCATTAA TATCAATTAT TGTTCCAACA TATAATGTTG AAAAATATAT TAGGACATGT 10200
ATTGAATCAA TCTTAGCTCA AACATATCGC AATATTGAAG TCATTATAGT AAATGATGGT 10260
AGCACAGATC AGTCGCTAGC AGTAATTTCC GATTTAATCT GTAGTCATCA TAATATTAAG 10320
GTAATCAACC AAAAAAACCA AGGATTATCA GTAGCTCGAA ACACTGGTAT TGATGCGGCA 10380
ACTGGTAAAT ATATAGCTTT TGTAGATGCA GATGACAAAA TTAAGCCAGA CTTTGTTAGC 10440
TCGCTCTATC AAATTGCTGA CAAAACAGGA GCTGACATTG TGCGTGGGTC ATTTCGAGAC 10500
TTTAATGGCA ATATTCCTAA AGGCTGGGTT CCAGATTTCA ATGTTCCAAC CAATTATGGG 10560
ACAATAGTAT TAGACCAATT CTTATCCAGC AACATATCTT TTGTAGTTTG GTCGAGTATC 10620
TATAGGCTAG ATTTTATTAA TAGTAATCAT ATACGATTTA CACCAGGGAT TCTATTTGAG 10680
GATGCAGATT TTACAATAAG AGCTTATATG CTCGCTAAGT TAGTTGCTAC ATCACCTGAA 10740
CCAAATTATG CATATAGAAT AAATCGTCCA GGAAGTATAT TAACCACAAA AACCACAAAA 10800
AATGCCCAAA AAATGTCTCT TTCAGAAGAA AAAATTATAT CACAATTTAT TAGTATGTTA 10860
AAGCATGAAA AATCTGATGT TTTATGTAGT TTAATTCTAA AGTCTATTTA TGCATGTATG 10920
AGAGATTGGA CGGGAATTAT TGTGAGGAAT AACCTATCGT TGGATAGGAA GAACAGTTGT 10980
TTTGATACTG CTCTCACTCT AATAAAAGAA ATAATAAATT CTAGGCCCTT AAAAGAAAAA 11040
ATCAAATTTT TAACAAAGGT TATTATTATT AAGGCGAAAA ACCATTAAGC CGTTAAACGA 11100
AAATCCAAAG GGTTCATATA CAATTATGTT AATTACGGAT TTTTTATATC TTCAATGGGT 11160
TCATTAATCA CTGAATTTGG TTATCTTGTT TTAATGAATG TGAAGTCATT CGGTTAAGGG 11220
GAGTCTTTGA TTTGTCTAGT TAGCTATCTG GACAGATGTT AAGTGTTAAT TACAGTGAAG 11280
GCAGATGAAA ACTTATTAAA AGTTATTCTG CTTGATTAAG AATGGTAAGA TTTCACCATC 11340
TATATACTTT TATTAGAACT TAGGTGGACA GGAGGACCCA ATTTTTAATC CTTCCTGTTA 11400
TATAGTTTTT GTTTAATATT TTTCGGGAGG ATTATTAATG CAAATAGTAA AAAATTATCT 11460
TTATAATGCA ATGTATCAGG TCTTTATAAT TATTGTGCCA TTACTTACCA TTCCTTATTT 11520
GTCAAGAATT TTGGGCCCTT CAGGTATTGG AATTAACTCA TATACCAATT CTATTGTTCA 11580
GTATTTTGTT TTATTTGGTA GTATAGGAGT CGGTTTGTAT GGGAATCGTC AGATTGCCTT 11640
TGTTAGGGAT AATCAGGTCA AAATGTCTAA AGTCTTTTAT GAAATATTTA TTTTAAGACT 11700
ATTAACAATA TGTTTAGCAT ATTTATTGTT CGTTGCTTTT TTAACCATTA ATGGTCAGTA 11760
TCATGAATAC TATTTGTTTC AATCCATTGC TATAGTTGCA GCTGCATTTG ATATCTCTTG 11820
GTTTTTTATG GGAATTGAAA ATTTTAAAGT AACTGTATTA AGAAATTTTA TAGTTCAGTT 11880
ACTTGCTCTA TTCAGTATTT TCCTATTTGT CAAATCTTAC AATGATTTGA ATATATATAT 11940
ATTGATAACA GTTTTATCTA CATTAATTGG TAATTTAACT TTTTTCCCAA GTTTACACAG 12000
ATATCTCGTA AAGGTTAACT ATCGTGAATT AAGGCCAATA AAGCATTTAA AGCAATCTTT 12060
AGTTATGTTT ATCCCACAAA TTGCTGTCCA AATTTATTGG GTTTTGAATA AAACAATGTT 12120

AGGTTCATTG GATTCTGTCA CGAGCTCCGG CTTTTTTGAT CAGTCTGATA AAATAGTTAA 12180
ACTGGTTTTG GCTATTGCTA GTGCAACAGG TACTGTCATG TTGCCACGTG TTGCAAATGC 12240
CTTTGCACAT AGAGAGTATA GTAAAATTAA GGAGTACATG TACGCAGGTT TTTCTTTTGT 12300
GTCGGCAATT TCGATTCCTA TGATGTTTGG TCTGATAGCT ATTACTCCTA AATTCGTGCC 12360
ACTTTTTTTT ACATCTCAAT TTAGTGATGT TATTCCTGTG TTAATGATCG AGTCAATTGC 12420
AATTATTTTT ATAGCTTGGA GCAACGCAAT AGGTAATCAA TATCTTTTAC CAACTAATCA 12480
AAATAAGTCA TATACAGTGT CGGTGTTCAT TGGAGCGATA GTCAATTTAA TGTTAAATAT 12540
TCCACTGATT ATATATCTAG GTGCTGTTGG TGCATCAATT GCAACTGTAA TTTCTGAAAT 12600
GTCTGTAACT GTGTATCAAC TTTTTATAAT TCATAAACAG CTTAATTTGC ATACACTGTT 12660
TTCGGATTTA TCTAAGTATT TAATTGCAGG ATTAGTGATG TTTCTAATTG TCTTTAAAAT 12720
TAGTTTGTTA ACACCGACAT CTTGGATATT CATTCTGTTG GAAATTACTG TGGGCATAAT 12780
TATTTATGTT GTTTTATTAA TATTTTTAAA GGCAGAAATA ATTAATAAAC TAAAGTTTAT 12840
TATGCATAAA TAGAGGTATG GATTTAGGTA CCTGCCTTTA GGATTTTTAA TTCAAAGGAT 12900
TTAGGTACTT ATGGTTACTT TAATTCGATT GTGACCTACT TTATTCTTTT GGCAACTTTA 12960
GGTGTTGCTA ACTATGGTAC TAAAGAGATT TCAGGACATC GAAAGGATAT TCGTAAAAAT 13020
TTCTGGGGTA TTTATACCCT CCAATTGATT GCGACTATTT TGTCTCTTGT CTTGTATACA 13080
TCATTATGTT TGTTCTTTCC TGGTATGCAA AATATGGTGG CTTATATCTT AGGATTAAGC 13140
TTGATATCGA AAGGAATGGA TATTTCTTGG TTATTCCAAG GTTTGGAGGA TTTTCGTCGT 13200
ATTACCGCAA GAATACAACG GTAAAGGTTT TAGGAGTTAT TTCTATCTTC CTATTTGTGA 13260
AAACACCTGG TGATTTGTAT CTCTATGTTT TCCTATTGAC CTTCTTTGAA TTGCTTGGGC 13320
AATTAAGTAT ATGGTTACCA GCGAGACCTT ACATTGGAAA ACCACAATTT GATTTATCCT 13380
ATGCTAAGAA ACATCTTAAA CCTGTTATTT TGCTGTTTCT CCCTCAGGTT GCCATTTCAC 13440
TATACGTGAC TTTGGATCGT ACAATGTTGG GTGCCTTGTC ATCGACAAAT GATGTAGGGA 13500
TTTATGATCA GGCTTTGAAA ATAATTAATA TTTTGTTGAC GTTGGTGACT TCATTGGGAA 13560
GTGTAATGCT TCCAAGGGTA TCTGGTCTTT TATCTAACGG AGATCATAAG GCCGTTAACA 13620
AGATGCATGA ATTCTCTTTC TTGATTTATA ATCTTGTGAT TTTCCCGATA ATAGCAGGTC 13680
TCTTGATTGT TAATAAGGAT TTTGTGAGTT TCTTACTAGG GAAAGATTTC CAAGAGGCTT 13740
ATCTTGCCAT TGCTATTATG GTCTTTAGGA TGTTCTTTAT CGGTTGGACA AATATTATGG 13800
GAATCCAGAT TTTGATTCCA TATAATAAAC ATCGTGAGTT CATGCTCTCT ACGACTATTC 13860
CGGCTGTTGT CAGTGTTGGA CTTAATCTCT TGTTAATTTC CTCCATTTGG CTTTGTTGGG 13920
GTCTCAATTG TATCAGTTTT AACAGAGGCT TTGGTATGGG TTATTCAATT GAATTTTTCA 13980
AGGATATTCA TCAAAGATGT GTCAATCCTT CCAGCCATGT CAAAAATTAT CTTAGCATCA 14040
GTTGTCATGT ATCTTGGACT CTTTGTCTTT AAGATGTTTG TGCAATTGAA ACCAATGCTA 14100
AATGTAGCAG TAGATGGTCT TGTCGGTGCT ATCATTTATA TTGTCTTGAT TATTGTCTTA 14160
CGTGTCGTTG ATATGAAAGA CTTGAAGCAA CAGTTAATGA AAAACTAAGG AGAAAAATAT 14220
GTACGATTAT CTTATCGTTG GTGCTGGTTT GTCCGGAGCA ATCTTCGCAC ATGAAGCTAC 14280
AAAACGTGGC AAAAAAGTAA AAGTGATTGA CAAGCGTGAT CACATTGGTG GCAATATCTA 14340
CTGTGAAGAT GTTGAAGGTA TTAATGTTCA CAAGTATGGT GCTCACATTT TCCATACCTC 14400
AAATAAAAAA GTTTGGGATT ATGTCAACCA ATTTGCTGAA TTTAATAACT ATATCAACTC 14460
ACCAATTGCT AACTACAAGG GCAGTCTTTA TAACCTTCCA TTTAACATGA ATACATTTTA 14520
TGCTATGTGG GGCACTAAGA CTCCTCAAGA AGTTAAGGAC AAGATTGCTG AACAAACGGC 14580
TGATATGAAA GATGTTGAGC CTAAAAACTT GGAAGAACAA GCTATCAAGT TGATTGGACC 14640
AGATATCTAC GAAAAGTTGA TCAAGGGATA CACTGAAAAA CAATGGGGAC GTTCTGCCAC 14700
AGACCTGCCT CCTTTCATCA TCAAGCGTCT TCCGGTTCGT CTAACTTTTG ATAACAACTA 14760
CTTTAATGAC CGTTACCAAG GAATTCCGAT CGGTGGTTAC AATGTCATCA TTGAAAATAT 14820
GCTTGGAGAT GTAGAAGTAG AGCTTGGAGT TGACTTCTTT GCCAATCGTG AAGAGCTTGA 14880
AGCTTCAGCT GAAAAAGTTG TCTTTACAGG AATGATTGAC CAGTACTTTG ATTATAAACA 14940
TGGTGAGTTG GAGTATCGCA GTCTTCGTTT TGAACACGAA GTCTTGGATG AAGAAAATCA 15000
TCAAGGAAAT GCCGTGGTCA ACTACACAGA GCGTGAGATT CCTTATACTC GTATCATTGA 15060
GCATAAGCAC TTCGAGTATG GTACACAACC TAAGACAGTT ATCACACGTG AATACCCAGC 15120
TGATTGGAAA CGTGGAGATG AACCATACTA CCCAATCAAT GATGAAAAGA ACAATGCCAT 15180
GTTTGCTAAG TACCAAGAAG AAGCTGAGAA AAATGACAAG GTTATCTTCT GTGGACGTCT 15240
TGCAGATTAT AAATACTACG ACATGCACGT GGTCATTGAG CGTGCTCTAG AAGTCGTTGA 15300
GAAAGAATTT ATTTAATAAA TAATGGCTCT TTGTCAACTG TAGTGGGTGA CGAAAAGCTA 15360
ACATCTAGAG AGGACCGGAT AGGTCCTTTT TTTATGTATG TTCAGYGTGA TGAAGACACG 15420
CTTCTTAAAG TTGATAAAGT TTCTAAAACC GAAGCCCAAA CGTTTGATGT CTTTAATCAA 15480
CTTGTTAGTC GCTTCAAGTT TCGCGTTTGA ATAGTCCGTT TCTAGTGCGT TTTTGATGTA 15540
TTGCTTGTGT CTAAGAAAAG TCCTAAAGAC AGTTTGAAAA TAATGATTGA CCTTGCTCCT 15600
ATTTTCCTCT ATCAGTTCAA AGAAATCATC TACTCTCTTC TCCTGAAAGT GAAAAAGCAA 15660
AAGCTGATAA AGTGTATAGT AGTCAGTAAG CTCTTTTGAA AAGACTAGTG TCTTCGCAAC 15720
AACTTCATGT GGTGCTAAAG TTTGGCGGAA AGTCTTTGAA TAAAAAGAAT TGAGAGATAG 15780
TTTACAGCTG TCCTTTTGGA AGAGTCGCCA GTGATTTTTC AAGGCTCGAT AGGGTGGTGA 15840
CTTCTTATCG AATTGGTTCA TGATTGCAAT TCTAGTCTTT AAAAATGCTC TACCAAGGTG 15900
CTGGATAATG TGGAAACGAT CAAGAACGAT TTTGGCGTTT GGAAAGAGTC TGCGGGATAG 15960
TGGGATATAG GCTCCAGACA TATCCATCGT GATAAACGGC ACCTGTTGTC GGACTTTCAA 16020
TGGATACTTC AAAAAGTAGT TTCGTATAGT AGTTTGGCGG CGATTATCAA GGATGATTAT 16080
GAGTTCGTTT GTCTCATACT TTTGAGCTAC AAAAGCCAAC TCTCCTTTCT TGAAACCAAA 16140
CTCATCCCAG GACATAACAG TAGGGAGTTT GTCATAATGT TCCTTGAAAG TAAACTGATC 16200

AAGCTTACAA TAGACAGTGG ACGTCGACAC GCGAAGTCTT CTTGCAATAT CAGTTAGTGA 16260
CACTTTCTCA GTTAGGAGTT GTGTAACTTT TTGTCGGACT AGATTGGAGA TTTGGCAGTT 16320
TTTCTCAACG ATAGATGTCT CAGCCACCGT TACTCTCCTA CAATTTTTAC ACTGGAAACG 16380
ACGTTTTTTC AGACGTAGTA GAGTTGGCGT TCCCGCTTGC TCGAGGAGAG GGATTTTAGA 16440
AGGCTTTTGA AAGTCGTACT TGATCATCTT TTCTTGGCAA TGAGGACATG ATGGTGCAGG 16500
GTAATCAAGT TTTGCTTGAA TCTCGATATG AGTGTCAGTT TCAAAAACAA GTGAAATAAT 16560
GATATTTTGG TCTTTAATTC CGATTAATTA TGTGGTATTC TTAATAGGTC TCATAAGTTC 16620
TTCCTAATGG TAGTTTCGTC GCTTTTCATT ATAGTTCTTA TGGGACTTTT TGTCTGCATT 16680
CAAAAAGCTC TATAATCTCT ACAATGGTTT TACCCACTAC AGAAATTATA GAGCCAATAT 16740
ATCTACTGTC TATTTTTATG CTACTTTTGG GTTAGCTCAA CTCAACCGCC TTTTAATCTC 16800
CCAACAACAA TAATACCCAA TTAAACAACC CAAGAAATTC AAGATAATAT CACTAATGGC 16860
AAATGTGCCC AAATAAAAGA TAAATTGAAT GGTTTCAATT CCTAAAAGTG TGACCAAACT 16920
GACAATGACA AACTGTTTGA AATCAGTATT GATACAGTAA AGGCCACCTA AAGGAATGAA 16980
GTAGATAATA TTTAGCACTG CCTCTTGAAT CGTTCTGGTA TCCGCTTTTA TAAAGTCAAA 17040
AGGATTAAGT GATATCGCCT GAAAATCCGT TGTTTTAGTA AAAAGCACCA TGAATAACAG 17100
TAATAAATAC ACACTGAAAG CAAGATAGAG ATAAATAACT GAAAAATGTT TGAGGTGATA 17160
CTGGATGCCA AACAACCAGA TAATCAGCGT TAATAAGAGT ATTAAAGTTA ATGTGGTATA 17220
GTCAAAGTGG TTAATCAACC TAGTCAGGCT TTGATAGCGA GTGAGA 17266

(2) INFORMATIONS POUR LA SEQ ID NO: 2:
(i) CARACTERISTIQUES DE LA SEQUENCE:
(A) LONGUEUR: 228 acides aminés
(B) TYPE: acide aminé
(C) NOMBRE DE BRINS: simple
(D) CONFIGURATION: linéaire
(ii) TYPE DE MOLECULE: protéine
(ix) CARACTERISTIQUE:
(A) NOM/CLE: Protein
(B) EMPLACEMENT:1..228
(D) AUTRES INFORMATIONS:/note= "EPS1"
(xi) DESCRIPTION DE LA SEQUENCE: SEQ ID NO: 2:

Met Asn Thr His Lys Ile Leu Leu Thr Thr Thr Pro Arg Asp Ala Tyr
1               5                   10                  15
Val Lys Ile Pro Gly Gly Gly Ala Asp Gln Tyr Asp Lys Leu Thr His
            20                  25                  30
Ala Gly Ile Tyr Gly Val Glu Thr Ser Glu Gln Thr Leu Glu Asp Leu
        35                  40                  45
Tyr Gly Thr Lys Ile Asp Tyr Tyr Ala Arg Ile Asn Phe Thr Ser Phe
    50                  55                  60
Leu Lys Leu Ile Asp Gln Leu Gly Gly Val Thr Val His Asn Asp Gln
65                  70                  75                  80
Ala Phe Thr Ser Leu His Gly Lys Phe Asp Phe Pro Val Gly Asp Ile
                85                  90                  95
Gln Met Asn Ser Glu Gln Ala Leu Gly Phe Val Arg Glu Arg Tyr Ser
            100                 105                 110
Leu Asp Gly Gly Asp Asn Asp Arg Gly Lys Asn Gln Glu Lys Val Ile
        115                 120                 125
Ser Ala Ile Val Asn Lys Leu Ala Ser Leu Lys Ser Val Ser Asn Phe
    130                 135                 140
Thr Ser Ile Val Asn Asn Leu Gln Asp Ser Val Gln Thr Asn Ile Ser
145                 150                 155                 160
Leu Asp Thr Ile Asn Ala Leu Ala Asn Thr Gln Leu Asp Ser Gly Ser
                165                 170                 175
Lys Phe Thr Val Thr Ser Gln Ala Val Thr Gly Thr Gly Ser Thr Gly
            180                 185                 190
Gln Leu Ile Ser Tyr Ala Met Pro Asn Ser Ser Leu Tyr Met Met Lys
        195                 200                 205
Leu Asp Asn Ser Ser Val Glu Ser Ala Ser Gln Ala Ile Lys Lys Leu
    210                 215                 220
Met Glu Glu Lys
225

(2) INFORMATIONS POUR LA SEQ ID NO: 3:
(i) CARACTERISTIQUES DE LA SEQUENCE:
(A) LONGUEUR: 243 acides aminés
(B) TYPE: acide aminé
(C) NOMBRE DE BRINS: simple
(D) CONFIGURATION: linéaire
(ii) TYPE DE MOLECULE: protéine
(ix) CARACTERISTIQUE:
(A) NOM/CLE: Protein
(B) EMPLACEMENT:1..243
(D) AUTRES INFORMATIONS:/note= "EPS2"
(xi) DESCRIPTION DE LA SEQUENCE: SEQ ID NO: 3:

Val Ile Asp Val His Ser His Ile Val Phe Asp Val Asp Asp Gly Pro
1               5                   10                  15
Lys Thr Leu Glu Glu Ser Leu Asp Leu Ile Gly Glu Ser Tyr Ala Gln
            20                  25                  30
Gly Val Arg Lys Ile Val Ser Thr Ser His Arg Arg Lys Gly Met Phe
        35                  40                  45
Glu Thr Pro Glu Asp Lys Ile Phe Ala Asn Phe Ser Lys Val Lys Ala
    50                  55                  60
Glu Ala Glu Glu Leu Tyr Pro Asp Leu Thr Ile Tyr Tyr Gly Gly Glu
65                  70                  75                  80
Leu Tyr Tyr Thr Ser Asp Ile Val Glu Lys Leu Glu Lys Asn Leu Ile
                85                  90                  95
Pro Arg Met His Asn Thr Gln Phe Ala Leu Ile Glu Phe Ser Ala Arg
            100                 105                 110
Thr Ser Trp Lys Glu Ile His Ser Gly Leu Ser Asn Val Leu Arg Ala
        115                 120                 125
Gly Val Thr Pro Ile Val Ala His Ile Glu Arg Tyr Asp Ala Leu Glu
    130                 135                 140
Glu Asn Ala Asp Arg Val Arg Glu Ile Ile Asn Met Gly Cys Tyr Thr
145                 150                 155                 160
Gln Val Asn Ser Ser His Val Leu Lys Pro Lys Leu Phe Gly Asp Lys
                165                 170                 175
Asp Lys Val Arg Lys Lys Arg Val Arg Phe Phe Leu Glu Lys Asn Leu
            180                 185                 190
Val His Met Val Ala Ser Asp Met His Asn Leu Gly Pro Arg Pro Pro
        195                 200                 205
Phe Met Lys Asp Ala Tyr Glu Ile Val Lys Lys Asn Tyr Gly Ser Lys
    210                 215                 220
Arg Ala Lys Asn Leu Phe Ile Glu Asn Pro Lys Thr Leu Leu Glu Asn
225                 230                 235                 240
Gln Tyr Leu

(2) INFORMATIONS POUR LA SEQ ID NO: 4:
(i) CARACTERISTIQUES DE LA SEQUENCE:
(A) LONGUEUR: 230 acides aminés
(B) TYPE: acide aminé
(C) NOMBRE DE BRINS: simple
(D) CONFIGURATION: linéaire
(ii) TYPE DE MOLECULE: protéine
(ix) CARACTERISTIQUE:
(A) NOM/CLE: Protéine
(B) EMPLACEMENT:1..230
(D) AUTRES INFORMATIONS:/note= "EPS3"
(xi) DESCRIPTION DE LA SEQUENCE: SEQ ID NO: 4:

Met Asn Gln Asp Asn Thr Lys Ser Asp Glu Ile Asp Val Leu Ala Leu
1               5                   10                  15
Leu His Lys Leu Trp Thr Lys Lys Leu Leu Ile Leu Phe Thr Ala Phe
            20                  25                  30
Tyr Phe Ala Ala Phe Ser Phe Leu Gly Thr Tyr Phe Phe Ile Gln Pro
        35                  40                  45

Thr Tyr Thr Ser Thr Thr Arg Ile Tyr Val Val Asn Gln Ala Thr Asp
    50                  55                  60
Asn Lys Asn Leu Ser Ala Glu Ala Leu Gln Ala Gly Thr Phe Leu Thr
65                  70                  75                  80
Lys Asp Tyr Lys Glu Ile Ile Thr Ser Asn Asp Val Leu Ser Glu Val
                85                  90                  95
Ile Lys Asp Glu Lys Leu Asn Met Thr Glu Ala Glu Leu Ala Lys Met
            100                 105                 110

Ile Ser Val Asp Ile Pro Thr Asp Thr Arg Leu Ile Leu Ile Ser Val
        115                 120                 125
Asn Ala Lys Thr Gly Gln Asp Ala Gln Thr Leu Ala Asn Lys Val Arg
    130                 135                 140
Glu Val Ala Ser Glu Lys Ile Lys Lys Val Thr Lys Val Glu Asp Val
145                 150                 155                 160
Thr Thr Leu Glu Glu Ala Lys Leu Pro Glu Ser Pro Ser Ser Pro Asn
                165                 170                 175
Ile Lys Leu Asn Val Leu Leu Gly Ala Val Leu Gly Gly Phe Leu Ala
            180                 185                 190
Val Ile Gly Val Leu Val Arg Glu Ile Leu Asp Asp Arg Val Arg Arg
        195                 200                 205
Pro Glu Asp Val Glu Asp Val Leu Gly Met Thr Leu Leu Gly Ile Ile
    210                 215                 220
Pro Asp Thr Asp Lys Ile
225                 230

(2) INFORMATIONS POUR LA SEQ ID NO: 5:
    (i) CARACTERISTIQUES DE LA SEQUENCE:
        (A) LONGUEUR: 232 acides aminés
        (B) TYPE: acide aminé
        (C) NOMBRE DE BRINS: simple
        (D) CONFIGURATION: linéaire
   (ii) TYPE DE MOLECULE: protéine
   (ix) CARACTERISTIQUE:
        (A) NOM/CLE: Protein
        (B) EMPLACEMENT:1..232
        (D) AUTRES INFORMATIONS:/note= "EPS4"
   (xi) DESCRIPTION DE LA SEQUENCE: SEQ ID NO: 5:

Met Pro Leu Leu Lys Leu Val Lys Ser Lys Val Asp Phe Ala Lys Lys
1               5                   10                  15
Thr Glu Glu Tyr Tyr Asn Ala Ile Arg Thr Asn Ile Gln Phe Ser Gly
            20                  25                  30
Ala Gln Ile Lys Val Ile Ala Ile Ser Ser Val Glu Ala Gly Glu Glu
        35                  40                  45
Lys Ser Thr Thr Ser Val Asn Leu Ala Ile Ser Phe Ala Ser Val Gly
    50                  55                  60
Leu Arg Thr Leu Leu Ile Asp Ala Asp Thr Arg Asn Ser Val Leu Ser
65                  70                  75                  80
Gly Thr Phe Lys Ser Asn Glu Pro Tyr Lys Gly Leu Ser Asn Phe Leu
                85                  90                  95
Ser Gly Asn Ala Asp Leu Asn Glu Thr Ile Cys Gln Thr Asp Ile Ser
            100                 105                 110
Gly Leu Asp Val Ile Ala Ser Gly Pro Val Ala Pro Asn Pro Thr Ser
        115                 120                 125
Leu Leu Gln Asn Asp Asn Phe Arg His Leu Met Glu Val Ala Arg Ser
    130                 135                 140
Arg Tyr Asp Tyr Val Ile Ile Asp Thr Pro Pro Ile Gly Leu Val Ile
145                 150                 155                 160
Asp Ala Val Ile Ile Ala His Gln Ala Asp Ala Ser Leu Leu Val Thr
                165                 170                 175
Ala Ala Gly Lys Ile Lys Arg Arg Phe Val Thr Lys Ala Val Glu Gln
            180                 185                 190
Leu Glu Gln Ser Gly Ser Gln Phe Leu Gly Val Val Leu Asn Lys Val
        195                 200                 205

Asp Met Thr Val Asp Lys Tyr Gly Ser Tyr Gly Ser Tyr Gly Ser Tyr
    210                 215                 220
Gly Glu Tyr Gly Lys Lys Ser Asp
225                 230

(2) INFORMATIONS POUR LA SEQ ID NO: 6:
    (i) CARACTERISTIQUES DE LA SEQUENCE:
        (A) LONGUEUR: 455 acides aminés
        (B) TYPE: acide aminé
        (C) NOMBRE DE BRINS: simple
        (D) CONFIGURATION: linéaire
    (ii) TYPE DE MOLECULE: protéine
    (ix) CARACTERISTIQUE:
        (A) NOM/CLE: Protein
        (B) EMPLACEMENT:1..455
        (D) AUTRES INFORMATIONS:/note= "EPS5"
    (xi) DESCRIPTION DE LA SEQUENCE: SEQ ID NO: 6:

Val Arg Glu Lys Gln Glu Ile Arg Arg Ile Glu Ile Gly Ile Ile Gln
1               5                   10                  15
Leu Val Val Val Val Phe Ala Ala Met Val Ala Ser Lys Ile Pro Tyr
            20                  25                  30
Thr Glu Ile Thr Gln Gly Ser Ile Val Leu Leu Gly Val Val His Val
        35                  40                  45
Val Ser Tyr Tyr Ile Ser Ser Tyr Tyr Glu Asn Leu Lys Tyr Arg Gly
    50                  55                  60
Tyr Leu Asp Glu Leu Ile Ala Thr Val Lys Tyr Cys Phe Ile Phe Ala
65                  70                  75                  80
Leu Ile Val Thr Phe Leu Ser Phe Phe Ala Asp Gly Ser Phe Ser Ile
                85                  90                  95
Ser Arg Arg Gly Leu Leu Tyr Val Thr Leu Ile Ser Gly Val Leu Leu
            100                 105                 110
Tyr Val Thr Asn Thr Val Leu Lys Tyr Phe Arg Ser Ser Ile Tyr Thr
        115                 120                 125
Arg Arg Lys Ser Asn Lys Asn Ile Leu Leu Ile Ser Asp Gln Ala Arg
    130                 135                 140
Leu Glu Asn Val Leu Ser Arg Met Lys Asp Asn Met Asp Gly Arg Ile
145                 150                 155                 160
Thr Ala Val Cys Val Leu Asp Asn Pro Tyr Phe Thr Asp Pro Phe Ile
                165                 170                 175
Lys Ser Val Lys Pro Glu Asn Leu Ile Glu Tyr Ala Thr His Ser Val
            180                 185                 190
Val Asp Gln Val Leu Ile Asn Leu Pro Ser Gly Gln Tyr Lys Ile Trp
        195                 200                 205
Asp Tyr Ala Ser Pro Phe Glu Ile Met Gly Ile Pro Val Ser Ile Asn
    210                 215                 220
Leu Asn Ala Leu Glu Phe Met Asn Gln Gly Glu Lys Arg Ile Gln Gln
225                 230                 235                 240
Leu Gly Pro Phe Lys Val Val Thr Phe Ser Thr Tyr Phe Tyr Ser Tyr
                245                 250                 255
Gly Asp Ile Leu Ala Lys Arg Phe Leu Asp Ile Cys Gly Ala Leu Val
            260                 265                 270
Gly Leu Leu Leu Cys Gly Ile Val Gly Ile Phe Leu Tyr Pro Leu Ile
        275                 280                 285
Arg Lys Asp Gly Gly Pro Ala Ile Phe Ala Gln Asp Arg Val Gly Glu
    290                 295                 300
Asn Gly Arg Ile Phe Lys Phe Tyr Lys Phe Arg Ser Met Cys Val Asp
305                 310                 315                 320
Ala Glu Glu Ile Lys Lys Asn Leu Met Ala Gln Asn Gln Met Ser Gly
                325                 330                 335
Gly Met Phe Lys Met Asp Asn Asp Pro Arg Ile Thr Lys Ile Gly His
            340                 345                 350
Phe Ile Arg Lys Thr Ser Leu Asp Glu Leu Pro Gln Phe Trp Asn Val
        355                 360                 365

Leu Lys Gly Asp Met Ser Leu Val Gly Thr Arg Pro Pro Thr Val Asp
    370                 375                 380
Glu Tyr Glu Lys Tyr Thr Pro Glu Gln Lys Arg Arg Leu Ser Phe Lys
385                 390                 395                 400
Pro Gly Ile Thr Gly Leu Trp Gln Val Ser Gly Arg Ser Glu Ile Thr
                405                 410                 415
Asp Phe Asp Glu Val Val Lys Leu Asp Val Ala Tyr Leu Asp Gly Trp
            420                 425                 430
Thr Ile Trp Arg Asp Ile Lys Ile Leu Leu Lys Thr Ile Lys Val Val
        435                 440                 445
Val Met Lys Asp Gly Ala Lys
    450                 455

(2) INFORMATIONS POUR LA SEQ ID NO: 7:
    (i) CARACTERISTIQUES DE LA SEQUENCE:
        (A) LONGUEUR: 681 acides aminés
        (B) TYPE: acide aminé
        (C) NOMBRE DE BRINS: simple
        (D) CONFIGURATION: linéaire
    (ii) TYPE DE MOLECULE: protéine
    (ix) CARACTERISTIQUE:
        (A) NOM/CLE: Protein
        (B) EMPLACEMENT:1..681
        (D) AUTRES INFORMATIONS:/note= "EPS6"
    (xi) DESCRIPTION DE LA SEQUENCE: SEQ ID NO: 7:

Met Lys Lys Ile Thr Ile Ala Val Ala Thr His Lys Lys Tyr Gln Met
1               5                   10                  15
Pro Lys Asp Asn Ile Tyr Leu Pro Ile Glu Cys Gly Ala Val Leu Arg
            20                  25                  30
Lys Asn His Leu Asp Tyr Ile Ala Asp Asp Ser Gly Asp Asn Ile Ser
        35                  40                  45
Glu Lys Asn Lys Asn Tyr Ser Glu Leu Thr Ala Leu Tyr Trp Leu Trp
    50                  55                  60
Lys Asn Asn Asp Ser Glu Tyr Lys Gly Leu Ala His Tyr Arg Arg His
65                  70                  75                  80
Phe Ser Asp Lys Lys Val Ser Ile Phe Ser Thr Gly Asn Phe Asp Asn
                85                  90                  95
Ile Leu Asp Arg Thr Val Leu Glu Glu His Leu Glu Lys Phe Asp Ile
            100                 105                 110
Ile Leu Pro Lys Leu Arg His Tyr Tyr Ile Glu Thr Ile Glu Ser His
        115                 120                 125
Tyr Lys His Thr His Phe Glu Lys Asp Leu Ile Ala Thr Glu Glu Ile
    130                 135                 140
Ile Lys Lys Leu Tyr Pro Asp Tyr Leu Asp Phe Tyr Tyr Ser Ala Leu
145                 150                 155                 160
Lys Arg Lys Ser Ala His Met Phe Asn Met Phe Ile Met Lys Asp Lys
                165                 170                 175
Tyr Phe Asn Asn Tyr Cys Glu Trp Leu Phe Ser Ile Leu Phe Glu Leu
            180                 185                 190
Glu Lys Val Leu Asp Ile Ser Glu Tyr Ser Pro Phe His Ala Arg Val
        195                 200                 205
Phe Gly Arg Val Ser Glu Ile Leu Leu Asp Val Trp Ile Phe Lys Asn
    210                 215                 220
Asn Leu Asn Phe Thr Glu Ile Pro Val Met Phe Met Glu Lys Gln Asn
225                 230                 235                 240
Trp Trp Asp Lys Ser Lys Arg Phe Ile Ser Ala Lys Leu Phe Asn Lys
                245                 250                 255

Lys Tyr Tyr Thr Arg Arg Asn Lys Leu Leu Thr Ile Gly Ile Ile Leu
            260                 265                 270
Ile Ile Phe Met Thr Ile Phe Asp Tyr Tyr Ile His Lys Thr Val Phe
        275                 280                 285
Ser Pro Val Phe Met Phe Asn Ser Leu Phe Leu Leu Ile Ile Ser Leu
    290                 295                 300
Ser Ser Met Arg Leu Tyr Asn Leu Arg Glu Tyr Ser Ile Lys Ser Ile
305                 310                 315                 320
Glu Val Ile Val Leu Gly Met Ile Phe Phe Ser Leu Gly Val Phe Cys
                325                 330                 335
Thr Arg Ile Val Ser His Glu Phe Leu Lys Asn Gln Asn Asn Val Ile
            340                 345                 350
Asn Tyr Asp Asp Asn Leu Asn Val Asn Trp Thr Phe Leu Lys Ile Leu
        355                 360                 365
Leu Ile Val Val Thr Thr Gly Asn Val Phe Ser Ile Ile Phe Ser Leu
    370                 375                 380
Lys Phe Leu Leu Gly Gly Gly Ser Tyr Leu Glu Leu Arg Asn Met Ile
385                 390                 395                 400
Leu Gly Tyr Asn Gly Ala Glu Pro Leu Ile Thr Asn Pro Leu Val Asn
                405                 410                 415
Ile Leu Thr Arg Tyr Ile Ser Gly Pro Gly Leu Thr Ala Leu Ile Pro
            420                 425                 430
Phe Ser Ile Phe Phe Leu Ile Arg Lys Lys Asn Ile Lys Phe Ser Leu
        435                 440                 445
Ile Ile Leu Leu Asn Leu Val Leu Ala Thr Leu Ser Ser Gly Gly Arg
    450                 455                 460
Ile Leu Leu Val Tyr Thr Ile Ile Gln Leu Phe Ile Gly Leu Ser Tyr
465                 470                 475                 480
Ser Lys Lys Asn Ile Pro Lys Lys Ile Lys Lys Val Val Ile Ile Ser
                485                 490                 495
Ser Ile Ile Phe Phe Ile Ser Ile Ile Val Leu Ser Asn Ile Arg Ser
            500                 505                 510
Ser Asn Ser Ile Tyr Arg Ala Phe Tyr Ala Tyr Phe Ser Gly Pro Val
        515                 520                 525
Val Leu Leu Ser Thr Trp Met Thr Asp Val Asp Thr Tyr Asn Ile His
    530                 535                 540
Ser His Gly Leu Gly Phe Ile Tyr Pro Ile Thr Tyr Leu Leu Asn Ser
545                 550                 555                 560
Phe Cys Asn Leu Ile Gly Ile Pro Asn Ser Met Leu Ala Asn Val Val
                565                 570                 575
Met Trp Gln Gly Met Pro Gln Asn Asp Trp Val Gly Val Phe Pro Asn
            580                 585                 590
Gln Ser Met Asn Ala Phe Ser Thr Leu Phe Tyr Phe Phe Tyr Lys Asp
        595                 600                 605
Phe Arg Glu Phe Gly Val Ala Cys Phe Ser Phe Leu Phe Gly Ser Ile
    610                 615                 620
Cys Gly Phe Ile Tyr Phe Lys Ala Phe Ile Glu Arg Lys Ser Lys Tyr
625                 630                 635                 640
Leu Val Tyr Tyr Leu Leu Gly Val Gln Ala Ile Ile Gly Ser Phe Ile
                645                 650                 655
Ile Trp Gln Leu Gly Ser Thr Ala Phe Phe Leu Ser Ile Val Phe Thr
            660                 665                 670
Ile Leu Ser Leu Lys Ser Lys Lys Ser
        675                 680

(2) INFORMATIONS POUR LA SEQ ID NO: 8:
(i) CARACTERISTIQUES DE LA SEQUENCE:
(A) LONGUEUR: 322 acides aminés
(B) TYPE: acide aminé
(C) NOMBRE DE BRINS: simple
(D) CONFIGURATION: linéaire
(ii) TYPE DE MOLECULE: protéine
(ix) CARACTERISTIQUE:
(A) NOM/CLE: Protein
(B) EMPLACEMENT:1..322
(D) AUTRES INFORMATIONS:/note= "EPS7"
(xi) DESCRIPTION DE LA SEQUENCE: SEQ ID NO: 8:

Met Thr Ile Ser Ile Val Ile Pro Val Tyr Asn Val Gln Asp Tyr Ile
1               5                   10                  15
Lys Lys Cys Leu Asp Ser Ile Leu Ser Gln Thr Phe Ser Asp Leu Glu
            20                  25                  30
Ile Ile Leu Val Asp Asp Gly Ser Thr Asp Leu Ser Gly Arg Ile Cys
        35                  40                  45
Asp Tyr Tyr Ser Glu Asn Asp Lys Arg Ile Lys Val Ile His Thr Ala
    50                  55                  60
Asn Gly Gly Gln Ser Glu Ala Arg Asn Val Gly Ile Lys Asn Ala Thr
65                  70                  75                  80
Ser Glu Trp Ile Thr Phe Ile Asp Ser Asp Asp Tyr Val Ser Ser Asp
                85                  90                  95
Tyr Ile Glu Tyr Leu Tyr Asn Leu Ile Gln Val His Asn Ala Asp Ile
            100                 105                 110
Ser Ile Ala Ser Phe Thr Tyr Ile Thr Pro Lys Lys Ile Ile Lys His
        115                 120                 125
Gly Asn Gly Glu Val Ala Leu Met Asp Ala Lys Thr Ala Ile Arg Arg
    130                 135                 140
Met Leu Leu Asn Glu Gly Phe Asp Met Gly Val Trp Gly Lys Met Tyr
145                 150                 155                 160
Arg Thr Glu Tyr Phe Asn Lys Tyr Lys Phe Val Ser Gly Lys Leu Phe
                165                 170                 175
Glu Asp Ser Leu Ile Thr Tyr Gln Ile Phe Ser Glu Ala Ser Thr Ile
            180                 185                 190
Val Phe Gly Ala Lys Asp Ile Tyr Phe Tyr Val Asn Arg Lys Asn Ser
        195                 200                 205
Thr Val Asn Gly Thr Phe Asn Ile Lys Lys Phe Asp Leu Ile Glu Met
    210                 215                 220
Asn Glu Glu Ala Asn Lys Phe Ile Lys His Lys Phe Pro Asp Leu Ser
225                 230                 235                 240
Ser Glu Ala His Arg Arg Met Ile Trp Ala Tyr Phe Ser Thr Leu Asn
                245                 250                 255
Gln Val Leu Ser Ser Thr Asn Glu His Asp Ile Asp Leu Tyr Ala Pro
            260                 265                 270
Gln Leu Val Ala Tyr Leu Leu Lys Gln Asp Lys Phe Ile Lys Arg Asn
        275                 280                 285
Thr Phe Ile Pro Lys Arg Asp Lys Ile Ala Phe Phe Ile Leu Lys Asn
    290                 295                 300
Phe Gly Leu Lys Thr Tyr Arg Asn Val Trp Asn Leu Tyr Leu Lys Met
305                 310                 315                 320
Thr Arg

(2) INFORMATIONS POUR LA SEQ ID NO: 9:
(i) CARACTERISTIQUES DE LA SEQUENCE:
(A) LONGUEUR: 316 acides aminés
(B) TYPE: acide aminé
(C) NOMBRE DE BRINS: simple
(D) CONFIGURATION: linéaire
(ii) TYPE DE MOLECULE: protéine

(ix) CARACTERISTIQUE:
(A) NOM/CLE: Protein
(B) EMPLACEMENT:1..316
(D) AUTRES INFORMATIONS:/note= "EPS8"
(xi) DESCRIPTION DE LA SEQUENCE: SEQ ID NO: 9:

Met Asn Pro Leu Ile Ser Ile Ile Val Pro Thr Tyr Asn Val Glu Lys
1               5                   10                  15
Tyr Ile Arg Thr Cys Ile Glu Ser Ile Leu Ala Gln Thr Tyr Arg Asn
            20                  25                  30
Ile Glu Val Ile Ile Val Asn Asp Gly Ser Thr Asp Gln Ser Leu Ala
        35                  40                  45
Val Ile Ser Asp Leu Ile Cys Ser His His Asn Ile Lys Val Ile Asn
    50                  55                  60
Gln Lys Asn Gln Gly Leu Ser Val Ala Arg Asn Thr Gly Ile Asp Ala
65                  70                  75                  80
Ala Thr Gly Lys Tyr Ile Ala Phe Val Asp Ala Asp Asp Lys Ile Lys
                85                  90                  95
Pro Asp Phe Val Ser Ser Leu Tyr Gln Ile Ala Asp Lys Thr Gly Ala
            100                 105                 110
Asp Ile Val Arg Gly Ser Phe Arg Asp Phe Asn Gly Asn Ile Pro Lys
        115                 120                 125
Gly Trp Val Pro Asp Phe Asn Val Pro Thr Asn Tyr Gly Thr Ile Val
    130                 135                 140
Leu Asp Gln Phe Leu Ser Ser Asn Ile Ser Phe Val Val Trp Ser Ser
145                 150                 155                 160
Ile Tyr Arg Leu Asp Phe Ile Asn Ser Asn His Ile Arg Phe Thr Pro
                165                 170                 175
Gly Ile Leu Phe Glu Asp Ala Asp Phe Thr Ile Arg Ala Tyr Met Leu
            180                 185                 190
Ala Lys Leu Val Ala Thr Ser Pro Glu Pro Asn Tyr Ala Tyr Arg Ile
        195                 200                 205
Asn Arg Pro Gly Ser Ile Leu Thr Thr Lys Thr Thr Lys Asn Ala Gln
    210                 215                 220
Lys Met Ser Leu Ser Glu Glu Lys Ile Ile Ser Gln Phe Ile Ser Met
225                 230                 235                 240
Leu Lys His Glu Lys Ser Asp Val Leu Cys Ser Leu Ile Leu Lys Ser
                245                 250                 255
Ile Tyr Ala Cys Met Arg Asp Trp Thr Gly Ile Ile Val Arg Asn Asn
            260                 265                 270
Leu Ser Leu Asp Arg Lys Asn Ser Cys Phe Asp Thr Ala Leu Thr Leu
        275                 280                 285
Ile Lys Glu Ile Ile Asn Ser Arg Pro Leu Lys Glu Lys Ile Lys Phe
    290                 295                 300
Leu Thr Lys Val Ile Ile Ile Lys Ala Lys Asn His
305                 310                 315

(2) INFORMATIONS POUR LA SEQ ID NO: 10:
(i) CARACTERISTIQUES DE LA SEQUENCE:
(A) LONGUEUR: 471 acides aminés
(B) TYPE: acide aminé
(C) NOMBRE DE BRINS: simple
(D) CONFIGURATION: linéaire
(ii) TYPE DE MOLECULE: protéine
(ix) CARACTERISTIQUE:
(A) NOM/CLE: Protein
(B) EMPLACEMENT:1..471
(D) AUTRES INFORMATIONS:/note= "EPS9"
(xi) DESCRIPTION DE LA SEQUENCE: SEQ ID NO: 10:

Met Gln Ile Val Lys Asn Tyr Leu Tyr Asn Ala Met Tyr Gln Val Phe
1               5                   10                  15
Ile Ile Ile Val Pro Leu Leu Thr Ile Pro Tyr Leu Ser Arg Ile Leu
            20                  25                  30

Gly Pro Ser Gly Ile Gly Ile Asn Ser Tyr Thr Asn Ser Ile Val Gln
        35                  40                  45
Tyr Phe Val Leu Phe Gly Ser Ile Gly Val Gly Leu Tyr Gly Asn Arg
    50                  55                  60
Gln Ile Ala Phe Val Arg Asp Asn Gln Val Lys Met Ser Lys Val Phe
65                  70                  75                  80
Tyr Glu Ile Phe Ile Leu Arg Leu Leu Thr Ile Cys Leu Ala Tyr Leu
                85                  90                  95
Leu Phe Val Ala Phe Leu Thr Ile Asn Gly Gln Tyr His Glu Tyr Tyr
            100                 105                 110
Leu Phe Gln Ser Ile Ala Ile Val Ala Ala Ala Phe Asp Ile Ser Trp
        115                 120                 125
Phe Phe Met Gly Ile Glu Asn Phe Lys Val Thr Val Leu Arg Asn Phe
    130                 135                 140
Ile Val Gln Leu Leu Ala Leu Phe Ser Ile Phe Leu Phe Val Lys Ser
145                 150                 155                 160
Tyr Asn Asp Leu Asn Ile Tyr Ile Leu Ile Thr Val Leu Ser Thr Leu
                165                 170                 175
Ile Gly Asn Leu Thr Phe Phe Pro Ser Leu His Arg Tyr Leu Val Lys
            180                 185                 190
Val Asn Tyr Arg Glu Leu Arg Pro Ile Lys His Leu Lys Gln Ser Leu
        195                 200                 205
Val Met Phe Ile Pro Gln Ile Ala Val Gln Ile Tyr Trp Val Leu Asn
    210                 215                 220
Lys Thr Met Leu Gly Ser Leu Asp Ser Val Thr Ser Ser Gly Phe Phe
225                 230                 235                 240
Asp Gln Ser Asp Lys Ile Val Lys Leu Val Leu Ala Ile Ala Ser Ala
                245                 250                 255
Thr Gly Thr Val Met Leu Pro Arg Val Ala Asn Ala Phe Ala His Arg
            260                 265                 270
Glu Tyr Ser Lys Ile Lys Glu Tyr Met Tyr Ala Gly Phe Ser Phe Val
        275                 280                 285
Ser Ala Ile Ser Ile Pro Met Met Phe Gly Leu Ile Ala Ile Thr Pro
    290                 295                 300
Lys Phe Val Pro Leu Phe Phe Thr Ser Gln Phe Ser Asp Val Ile Pro
305                 310                 315                 320
Val Leu Met Ile Glu Ser Ile Ala Ile Ile Phe Ile Ala Trp Ser Asn
                325                 330                 335
Ala Ile Gly Asn Gln Tyr Leu Leu Pro Thr Asn Gln Asn Lys Ser Tyr
            340                 345                 350
Thr Val Ser Val Phe Ile Gly Ala Ile Val Asn Leu Met Leu Asn Ile
        355                 360                 365
Pro Leu Ile Ile Tyr Leu Gly Ala Val Gly Ala Ser Ile Ala Thr Val
    370                 375                 380
Ile Ser Glu Met Ser Val Thr Val Tyr Gln Leu Phe Ile Ile His Lys
385                 390                 395                 400
Gln Leu Asn Leu His Thr Leu Phe Ser Asp Leu Ser Lys Tyr Leu Ile
                405                 410                 415
Ala Gly Leu Val Met Phe Leu Ile Val Phe Lys Ile Ser Leu Leu Thr
            420                 425                 430
Pro Thr Ser Trp Ile Phe Ile Leu Leu Glu Ile Thr Val Gly Ile Ile
        435                 440                 445
Ile Tyr Val Val Leu Leu Ile Phe Leu Lys Ala Glu Ile Ile Asn Lys
    450                 455                 460
Leu Lys Phe Ile Met His Lys
465                 470

(2) INFORMATIONS POUR LA SEQ ID NO: 11:
    (i) CARACTERISTIQUES DE LA SEQUENCE:
        (A) LONGUEUR: 365 acides aminés
        (B) TYPE: acide aminé
        (C) NOMBRE DE BRINS: simple
        (D) CONFIGURATION: linéaire
    (ii) TYPE DE MOLECULE: protéine

(ix) CARACTERISTIQUE:
(A) NOM/CLE: Protein
(B) EMPLACEMENT:1..365
(D) AUTRES INFORMATIONS:/note= "EPS10"
(xi) DESCRIPTION DE LA SEQUENCE: SEQ ID NO: 11:

Met Tyr Asp Tyr Leu Ile Val Gly Ala Gly Leu Ser Gly Ala Ile Phe
1               5                   10                  15
Ala His Glu Ala Thr Lys Arg Gly Lys Lys Val Lys Val Ile Asp Lys
            20                  25                  30
Arg Asp His Ile Gly Gly Asn Ile Tyr Cys Glu Asp Val Glu Gly Ile
        35                  40                  45
Asn Val His Lys Tyr Gly Ala His Ile Phe His Thr Ser Asn Lys Lys
    50                  55                  60
Val Trp Asp Tyr Val Asn Gln Phe Ala Glu Phe Asn Asn Tyr Ile Asn
65                  70                  75                  80
Ser Pro Ile Ala Asn Tyr Lys Gly Ser Leu Tyr Asn Leu Pro Phe Asn
                85                  90                  95
Met Asn Thr Phe Tyr Ala Met Trp Gly Thr Lys Thr Pro Gln Glu Val
            100                 105                 110
Lys Asp Lys Ile Ala Glu Gln Thr Ala Asp Met Lys Asp Val Glu Pro
        115                 120                 125
Lys Asn Leu Glu Glu Gln Ala Ile Lys Leu Ile Gly Pro Asp Ile Tyr
    130                 135                 140
Glu Lys Leu Ile Lys Gly Tyr Thr Glu Lys Gln Trp Gly Arg Ser Ala
145                 150                 155                 160
Thr Asp Leu Pro Pro Phe Ile Ile Lys Arg Leu Pro Val Arg Leu Thr
                165                 170                 175
Phe Asp Asn Asn Tyr Phe Asn Asp Arg Tyr Gln Gly Ile Pro Ile Gly
            180                 185                 190
Gly Tyr Asn Val Ile Ile Glu Asn Met Leu Gly Asp Val Glu Val Glu
        195                 200                 205
Leu Gly Val Asp Phe Phe Ala Asn Arg Glu Glu Leu Glu Ala Ser Ala
    210                 215                 220
Glu Lys Val Val Phe Thr Gly Met Ile Asp Gln Tyr Phe Asp Tyr Lys
225                 230                 235                 240
His Gly Glu Leu Glu Tyr Arg Ser Leu Arg Phe Glu His Glu Val Leu
                245                 250                 255
Asp Glu Glu Asn His Gln Gly Asn Ala Val Val Asn Tyr Thr Glu Arg
            260                 265                 270
Glu Ile Pro Tyr Thr Arg Ile Ile Glu His Lys His Phe Glu Tyr Gly
        275                 280                 285
Thr Gln Pro Lys Thr Val Ile Thr Arg Glu Tyr Pro Ala Asp Trp Lys
    290                 295                 300
Arg Gly Asp Glu Pro Tyr Tyr Pro Ile Asn Asp Glu Lys Asn Asn Ala
305                 310                 315                 320
Met Phe Ala Lys Tyr Gln Glu Glu Ala Glu Lys Asn Asp Lys Val Ile
                325                 330                 335
Phe Cys Gly Arg Leu Ala Asp Tyr Lys Tyr Tyr Asp Met His Val Val
            340                 345                 350
Ile Glu Arg Ala Leu Glu Val Val Glu Lys Glu Phe Ile
        355                 360                 365

(2) INFORMATIONS POUR LA SEQ ID NO: 12:
(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "amorces"
(xi) DESCRIPTION DE LA SEQUENCE: SEQ ID NO: 12:

GAYGARYTNC CNCARYTNWK NAAYGT

(2) INFORMATIONS POUR LA SEQ ID NO: 13:
(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "amorces"
(xi) DESCRIPTION DE LA SEQUENCE: SEQ ID NO: 13:

CCANHRNCCN GTNATNCCNG GYTT 24

## Revendications

1. Enzyme recombinée, susceptible d'être impliquée dans la biosynthèse de l'EPS ayant l'unité saccharidique répétitive 
β-D-Galp1
↓
6
→3)-β-D-Glcp-(1→3)-β-D-Galf-(1→3)-α-D-Glcp-(1→
 catalysant spécifiquement l'une des liaisons suivantes entre sucres:
- la liaison osidique β-1,3 entre le carbone 1 d'un D-Gal*f* activé et le carbone 3 du Glc*p* présent à l'extrémité non-réductrice d'une chaîne saccharidique en formation ;
- la liaison osidique β-1,3 entre le carbone 1 d'un D-Glc*p* activé et le carbone 3 du Gal*f* présent à l'extrémité non-réductrice d'une chaîne saccharidique en formation ;
- la liaison osidique β-1,6 entre le carbone 1 d'un D-Gal*p* activé et le carbone 6 du Glc*p* présent à l'extrémité non-réductrice d'une chaîne saccharidique en formation ; et,
- la liaison osidique α-1,3 entre les unités saccharidiques répétitives de l'EPS ci-dessus.

2. Enzyme recombinée, notamment selon la revendication 1, ayant l'une des séquences en acides aminés choisie dans le groupe formé par les séquences SEQ ID NO:7, SEQ ID NO:8, SEQ ID NO:9, SEQ ID NO:10, SEQ ID NO:11, et les séquences qui leur sont homologues.

3. Enzyme recombinée, notamment selon la revendication 1, ayant l'une des séquences en acides aminés choisie dans le groupe formé par les séquences SEQ ID NO:2, SEQ ID NO:3, SEQ ID NO:4, SEQ ID NO:5 et SEQ ID NO:6.

4. ADN recombiné codant pour une enzyme recombinée selon l'une des revendications 2 à 3.

5. ADN selon la revendication 4, comprenant au moins un gène ayant une séquence nucléotidique choisie parmi les séquences délimitées dans la séquence SEQ ID NO:1 par les nucléotides 5933-7978, 8003-8969, 10138-11086, 11438-12852, 14220-15315, et les séquences qui leur sont homologues.

6. ADN selon la revendication 4, comprenant au moins un gène ayant une séquence nucléotidique choisie parmi les séquences délimitées dans la séquence SEQ ID NO:1 par les nucléotides 1-687, 690-1418, 1429-2119, 2131-2870 et 2929-4244.

7. Vecteur recombinant comprenant un ADN selon l'une des revendication 4-6.

8. Cellule comprenant, intégré dans son génome ou par le moyen d'un plasmide réplicable, un ADN recombiné selon l'une des revendication 4 à 6, et exprimant une enzyme recombinée fonctionnelle selon l'une des revendications 1 à 3.

9. Cellule selon la revendication 9, caractérisée en ce qu'il s'agit d'une bactérie lactique.

10. Procédé de production d'un EPS, dans lequel (1) on clone dans un vecteur un fragment d'ADN codant pour au moins l'une des enzymes selon l'une des revendications 1 à 3, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

11. Procédé selon la revendication 10, dans lequel le vecteur comprend en outre au moins une séquence promoteur fonctionnelle et au moins une séquence d'activation traductionnelle fonctionnelle, permettant l'expression des ADN codant pour au moins l'une des enzymes selon l'une des revendications 1 à 3.

12. Utilisation d'une enzyme recombinée selon l'une des revendications 1 à 3, ou d'un ADN selon l'une des revendication 4 à 6, pour la synthèse d'un EPS.
